# EUROPEAN PATENT APPLICATION

(11) **EP 1 930 325 A1**
(43) Date of publication of application: **11.06.2008**
(21) Application number: 06810980.0
(22) Date of filing: 25.09.2006
(51) Int. Cl.: C07D 241/04, C07B 51/00, C07B 53/00, C07C 231/12, C07C 269/06, C07C 271/22, C07D 241/08, C07D 401/04, C07D 471/14

(54) **PROCESS FOR PRODUCING OPTICALLY ACTIVE PIPERAZINE COMPOUND**

(30) Priority: 26.09.2005 JP 2005278758; 22.03.2006 JP 2006079486
(71) Applicant: Sumitomo Chemical Company, Limited, Tokyo 104-8260 (JP)
(72) Inventor: MAEDA, Hiroshi, Osaka-shi, Osaka 555-0021 (JP); MATSUI, Kozo, Kobe-shi, Hyogo 650-0046 (JP); ITAYA, Nobushige, Nishinomiya-shi, Hyogo 662-0841 (JP)
(74) Representative: Duckworth, Timothy John
(86) International application number: PCT/JP2006/319625
(87) International publication number: WO 2007/035003

(57) **Abstract**

Provided is a method of producing optically active 1-methyl-3-phenylpiperazine of the formula (11) or salt thereof, comprising the following steps 1 to 4, or steps 5 to 7 and step 4, and a method of producing optically active mirtazapine via this method.

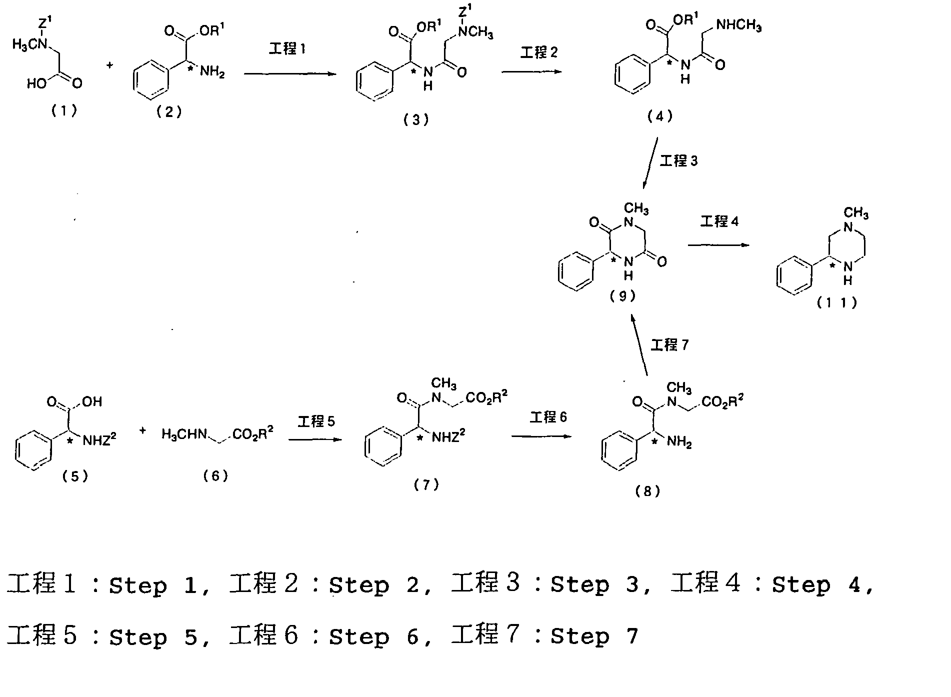

## Description

### Technical Field

The present invention relates to novel optically active 1-methyl-3-phenylpiperazine and salts thereof which are useful for production of pharmaceutical products such as optically active mirtazapine and the like, and to production methods thereof and intermediates thereof, and to production methods of optically active 1-(3-hydroxymethyl-2-pyridyl)-4-methyl-2-phenylpiperazine and salts thereof, and to intermediates thereof.

### Background Art

Mirtazapine is a drug useful as an antidepressant. Recently, optically active mirtazapine is being developed as a therapeutic agent for insomnia and the like, and a production method from (S)-1-(3-hydroxymethyl-2-pyridyl)-4-methyl-2-phenylpiperazine is disclosed in WO 2005/005410.

WO 2005/005410 discloses that optically active 1-(3-hydroxymethyl-2-pyridyl)-4-methyl-2-phenylpiperazine can be produced by methods such as an asymmetric synthesis method, an optical resolution method with a chiral acid of a racemate, and the like, however, there is no disclosure with respect to specific production thereof.

Meanwhile, it is known that racemic 1-(3-hydroxymethyl-2-pyridyl)-4-methyl-2-phenylpiperazine can be produced from racemic 1-methyl-3-phenylpiperazine via racemic 2-(4-methyl-2-phenylpiperazinyl)-3-pyridine carboxylic acid (for example, US 4062848, US 6852855 and the like).

Additionally, it is known that racemic 1-methyl-3-phenylpiperazine can be produced from N-(2-hydroxyethyl)-N-methyl-α-hydroxy-β-phenethylamine (for example, US 6495685 and the like).

Further, it is also known that racemic 1-(3-hydroxymethyl-2-pyridyl)-4-methyl-2-phenylpiperazine can be produced from racemic 1-methyl-3-phenylpiperazine via racemic 2-(4-methyl-2-phenylpiperazin-1-yl)-3-pyridinecarbaldehyde (for example, JP 2001-122874-A and the like).

Though the above-described literatures disclose racemic 1-methyl-3-phenylpiperazine, optically active substances thereof are not disclosed in any of the literatures at all. It is hypothesized that the optically active substance is obtained by optical resolution of a racemate, however, in this method, an undesirable half enantiomer is formed, leading to an economical disadvantage. There is also a case of adopting a means for recovery by racemization of the undesirable enantiomer, however, this means results in increase of the number of steps, and the like, also leading to an economical disadvantage.

A simple and economically advantageously method for producing optically active 1-methyl-3-phenylpiperazine is desired for economically advantageous production of pharmaceutical products such as optically active mirtazapine and the like.

On the other hand, the present inventors have tried production of optically active 1-(3-hydroxymethyl-2-pyridyl)-4-methyl-2-phenylpiperazine from optically active 1-methyl-3-phenylpiperazine by the same methods as described in US 4062848 and US 6852855, to find occurrence of isomerization without retaining its steric configuration.

A method for producing optically active 1-(3-hydroxymethyl-2-pyridyl)-4-methyl-2-phenylpiperazine from optically active 1-methyl-3-phenylpiperazine while retaining steric configuration is desired for advantageous production of pharmaceutical products such as optically active mirtazapine and the like.

### DISCLOSURE OF THE INVENTION

The present invention has an object of providing optically active 1-methyl-3-phenylpiperazine and salts thereof which are useful for production of pharmaceutical products such as optically active mirtazapine and the like, production methods thereof and intermediates thereof, and production methods of optically active 1-(3-hydroxymethyl-2-pyridyl)-4-methyl-2-phenylpiperazine and salts thereof, and intermediates thereof.

The present inventors have intensively studied the above-described problems, leading to completion of the present invention.

That is, the present invention is as described below.
<1> A method of producing optically active 1-methyl-3-phenylpiperazine of the formula (11): 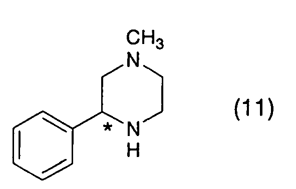 (wherein, a carbon atom with * represents an asymmetrical carbon atom.)
   (hereinafter, referred to as optically active 1-methyl-3-phenylpiperazine (11) in some cases.)
   or salt thereof, comprising the following steps 1 to 4:
   (step 1)
   a sarcosine compound of the formula (1): 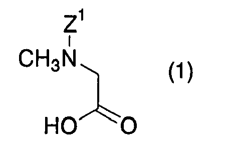 (wherein, Z¹ represents an amino group-protective group.) (hereinafter, referred to as sarcosine compound (1) in some cases.)
   or a reactive derivative at its carboxyl group is reacted with an optically active phenylglycine compound of the formula (2): 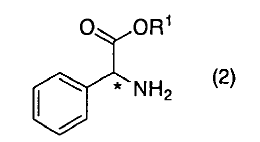 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and * has the same definition as described above.)
   (hereinafter, referred to as optically active phenylglycine compound (2) in some cases.)
   to produce an optically active compound of the formula (3): 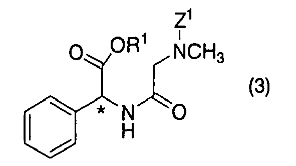 (wherein, Z¹, R¹ and * are as defined above.)
   (hereinafter, referred to as optically active compound (3) in some cases.)
   or salt thereof;
   (step 2)
   the protective group Z¹ is removed from the optically active compound (3) or salt thereof to produce an optically active compound of the formula (4): 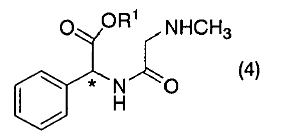 (wherein, marks are as defined above.)
   (hereinafter, referred to as optically active compound (4) in some cases.)
   or salt thereof;
   (step 3)
   the optically active compound (4) or salt thereof is cyclized to produce an optically active 1-methyl-3-phenylpiperazine-2,5-dione of the formula (9): 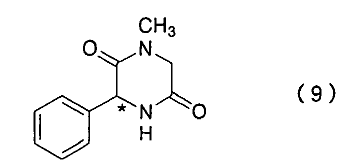 (hereinafter, referred to as optically active 1-methyl-3-phenylpiperazine-2,5-dione (9) in some cases.)
   or salt thereof;
   (step 4)
   the 1-methyl-3-phenylpiperazine-2,5-dione (9) or salt thereof is reduced to produce optically active 1-methyl-3-phenylpiperazine (11) or salt thereof.
<2> The method according to <1>, wherein the step 1 is carried out by reacting a reactive derivative at a carboxyl group of a sarcosine compound (1) with an optically active phenylglycine compound (2), or reacting a sarcosine compound (1) with an optically active phenylglycine compound (2) in the presence of a condensation agent,
   Z¹ is an optionally substituted benzyloxycarbonyl group and removal in the step 2 is carried out by catalytic reduction, or Z¹ is a tert-butoxycarbonyl group and the removal is carried out by an acid treatment,
   cyclization in the step 3 is carried out by at least one treatment selected from the group consisting of a heat treatment, pressure reduction treatment, base treatment and acid treatment, and
   reduction in the step 4 is carried out by reacting an optically active 1-methyl-3-phenylpiperazine-2,5-dione (9) or salt thereof with a reducing agent.
<3> The method according to <1> or <2>, wherein the optically active phenylglycine compound (2) is an S-isomer and the optically active 1-methyl-3-phenylpiperazine (11) or salt thereof is an S-isomer.
<4> A method of producing an optically active 1-methyl-3-phenylpiperazine (11) or salt thereof, comprising the following steps 5 to 7 and the step 4:
   (step 5)
   an optically active phenylglycine compound of the formula (5): 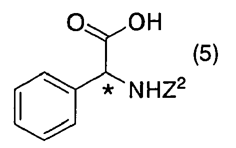 (wherein, Z² represents an amino group-protective group, and * has the same definition as described above.)
   (hereinafter, referred to as optically active phenylglycine compound (5) in some cases.)
   or a reactive derivative at its carboxyl group is reacted with a sarcosine compound of the formula (6): 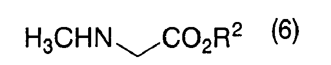 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms.)
   (hereinafter, referred to as sarcosine compound (6) in some cases.)
   to produce an optically active compound of the formula (7): 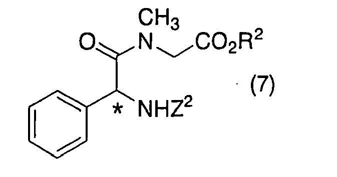 (wherein, Z², R² and * have the same definitions as defined above.)
   (hereinafter, referred to as optically active compound (7) in some cases.)
   or salt thereof;
   (step 6)
   the protective group Z² is removed from an optically active compound (7) or salt thereof to produce an optically active compound of the formula (8): 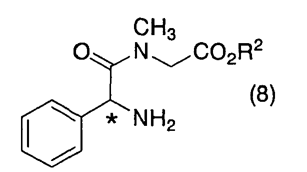 (wherein, R² and * have the same meanings as defined above.)
   (hereinafter, referred to as optically active compound (8) in some cases.)
   or salt thereof;
   (step 7)
   the optically active compound (8) or salt thereof is cyclized to produce optically active 1-methyl-3-phenylpiperazine-2,5-dione (9) or salt thereof;
   (step 4)
   the 1-methyl-3-phenylpiperazine-2,5-dione (9) is reduced to produce optically active 1-methyl-3-phenylpiperazine (11) or salt thereof.
<5> The method according to <4>, wherein the step 5 is carried out by reacting a reactive derivative at a carboxyl group of an optically active phenylglycine compound (5) with a sarcosine compound (6), or reacting an optically active phenylglycine compound (5) with a sarcosine compound (6) in the presence of a condensation agent,
   Z² is an optionally substituted benzyloxycarbonyl group and removal in the step 6 is carried out by catalytic reduction, or Z² is a tert-butoxycarbonyl group and the removal is carried out by an acid treatment,
   cyclization in the step 7 is carried out by at least one treatment selected from the group consisting of a heat treatment, pressure reduction treatment, base treatment and acid treatment, and
   reduction in the step 4 is carried out by allowing a reducing agent to act on the optically active 1-methyl-3-phenylpiperazine-2,5-dione (9) or salt thereof.
<6> The method according to <4> or <5>, wherein the optically active phenylglycine compound (5) is an S-isomer and the optically active 1-methyl-3-phenylpiperazine (11) or salt thereof is an S-isomer.
<7> A method of producing optically active mirtazapine of the formula (15): 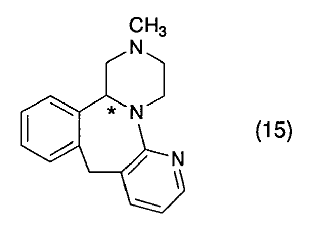 (wherein, a carbon atom with * represents an asymmetrical carbon atom.)
   (hereinafter, referred to as optically active mirtazapine (15) in some cases.)
   or salt thereof, comprising the following steps 11 to 14:
   (step 11)
   optically active 1-methyl-3-phenylpiperazine (11) or salt thereof is condensed with 2-halogeno-3-cyano-pyridine in the presence of a base to produce optically active 1-(3-cyano-2-pyridyl)-4-methyl-2-phenylpiperazine of the formula (12): 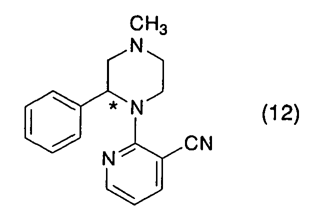 (wherein, * has the same definition as described above.) (hereinafter, referred to as optically active 1-(3-cyano-2-pyridyl)-4-methyl-2-phenylpiperazine (12) in some cases.)
   or salt thereof;
   (step 12)
   The optically active 1-(3-cyano-2-pyridyl)-4-methyl-2-phenylpiperazine (12) or salt thereof is reduced with a reducing agent to produce optically active 2-(4-methyl-2-phenylpiperazin-1-yl)-3-pyridinecarbaldehyde of the formula (13): 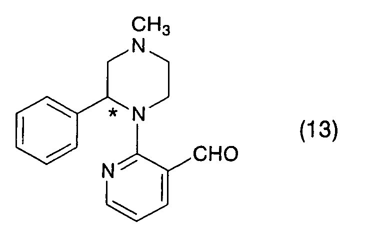 (wherein, * has the same definition as described above.)
   (hereinafter, referred to as optically active 2-(4-methyl-2-phenylpiperazin-1-yl)-3-pyridinecarbaldehyde (13) in some cases.)
   or salt thereof;
   (step 13)
   The optically active 2-(4-methyl-2-phenylpiperazin-1-yl)-3-pyridinecarbaldehyde (13) or salt thereof is reduced with a reducing agent to produce optically active 1-(3-hydroxymethyl-2-pyridyl)-4-methyl-2-phenylpiperazine of the formula (14): 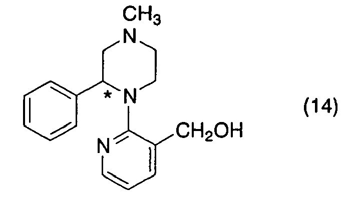 (wherein, * has the same definition as described above.)
   (hereinafter, referred to as optically active 1-(3-hydroxymethyl-2-pyridyl)-4-methyl-2-phenylpiperazine (14) in some cases.)
   or salt thereof;
   (step 14)
   The optically active 1-(3-hydroxymethyl-2-pyridyl)-4-methyl-2-phenylpiperazine (14) or salt thereof is cyclized to produce optically active mirtazapine (15) or salt thereof.
<8> The method according to <7>, wherein,
   in the step 11, the 2-halogeno-3-cyano-pyridine is 2-chloro-3-cyano-pyridine or 2-bromo-3-cyano-pyridine and the base is an organic base,
   in the step 12, the reducing agent is hydrogenated diisobutylaluminum, hydrogenated diisopropylaluminum or hydrogenated dihexylaluminum,
   in the step 13, the reducing agent is sodium borohydride, sodium cyanoborohydride or lithium borohydride, and
   in the step 14, the cyclization is carried out by an acid treatment.
<9> The method according to <7> or <8>, wherein the optically active 1-methyl-3-phenylpiperazine (11) or salt thereof is an S-isomer, and the optically active mirtazapine (15) or salt thereof is an S-isomer.
<10> The method according to any one of <7> to <9>, wherein the optically active 1-methyl-3-phenylpiperazine (11) or salt thereof is obtained by the following steps 1 to 4:
   (step 1)
   a sarcosine compound of the formula (1) or a reactive derivative at its carboxyl group is reacted with an optically active phenylglycine compound of the formula (2) to produce an optically active compound (3) or salt thereof;
   (step 2)
   the protective group Z¹ is removed from the optically active compound (3) or salt thereof to produce an optically active compound (4) or salt thereof;
   (step 3)
   the optically active compound (4) or salt thereof is cyclized to produce an optically active 1-methyl-3-phenylpiperazine-2,5-dione (9) or salt thereof;
   (step 4)
   the optically active 1-methyl-3-phenylpiperazine-2,5-dione (9) or salt thereof is reduced to produce optically active 1-methyl-3-phenylpiperazine (11) or salt thereof.
<11> The method according to any one of <7> to <9>, wherein the optically active 1-methyl-3-phenylpiperazine (11) or salt thereof is obtained by the following steps 5 to 7 and the step 4:
   (step 5)
   an optically active phenylglycine compound (5) or a reactive derivative at its carboxyl group is reacted with a sarcosine compound (6) to produce an optically active compound
   (7) or salt thereof;
   (step 6)
   the protective group Z² is removed from the optically active compound (7) or salt thereof to produce an optically active compound (8) or salt thereof;
   (step 7)
   the optically active compound (8) or salt thereof is cyclized to produce optically active 1-methyl-3-phenylpiperazine-2,5-dione (9) or salt thereof;
   (step 4)
   the optically active 1-methyl-3-phenylpiperazine-2,5-dione (9) is reduced to produce optically active 1-methyl-3-phenylpiperazine (11) or salt thereof.
<12> A method of producing optically active 1-(3-hydroxymethyl-2-pyridyl)-4-methyl-2-phenylpiperazine (14) or salt thereof, comprising the following steps 11 to 13:
   (step 11)
   optically active 1-methyl-3-phenylpiperazine (11) or salt thereof is condensed with 2-halogeno-3-cyano-pyridine in the presence of a base to produce optically active 1-(3-cyano-2-pyridyl)-4-methyl-2-phenylpiperazine of the formula (12) or salt thereof;
   (step 12)
   the optically active 1-(3-cyano-2-pyridyl)-4-methyl-2-phenylpiperazine (12) or salt thereof is reduced with a reducing agent to produce optically active 2-(4-methyl-2-phenylpiperazin-1-yl)-3-pyridinecarbaldehyde (13) or salt thereof;
   (step 13)
   the optically active 2-(4-methyl-2-phenylpiperazin-1-yl)-3-pyridinecarbaldehyde (13) or salt thereof is reduced with a reducing agent to produce optically active 1-(3-hydroxymethyl-2-pyridyl)-4-methyl-2-phenylpiperazine of the formula (14) or salt thereof.
<13> Optically active 1-methyl-3-phenylpiperazine (11) or salt thereof.
<14> The compound according to <13>, wherein the compound is an S-isomer.
<15> 1-methyl-3-phenylpiperazine-2,5-dione of the formula (9'): 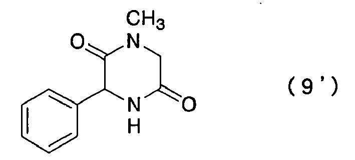 (hereinafter, referred to as 1-methyl-3-phenylpiperazine-2,5-dione (9') in some cases.).
<16> The compound according to <15>, wherein the compound is an S-isomer.
<17> An optically active compound (3).
<18> The compound according to <17>, wherein the compound is an S-isomer.
<19> An optically active compound (4) or salt thereof.
<20> The compound according to <19> or salt thereof, wherein the compound is an S-isomer.
<21> An optically active compound (7) or salt thereof.
<22> The compound according to <21>, wherein the compound is an S-isomer.
<23> An optically active compound (8) or salt thereof.
<24> The compound according to <23>, wherein the compound is an S-isomer.
<25> Optically active 1-(3-cyano-2-pyridyl)-4-methyl-2-phenylpiperazine (12) or salt thereof.
<26> The compound according to <25>, wherein the compound is an S-isomer.
<27> Optically active 2-(4-methyl-2-phenylpiperazin-1-yl)-3-pyridinecarbaldehyde (13) or salt thereof.
<28> The compound according to <27>, wherein the compound is an S-isomer.
<29> A method of producing optically active 1-methyl-3-phenylpiperazine (11) or salt thereof, comprising reducing optically active 1-methyl-3-phenylpiperazine-2,5-dione (9).
<30> The method according to <29>, wherein reduction is carried out by reacting optically active 1-methyl-3-phenylpiperazine-2,5-dione (9) or salt thereof with a metal hydride compound.
<31> The method according to <30>, wherein the metal hydride compound is lithium aluminum hydride.
<32> The method according to any one of <29> to <32>, wherein both the optically active 1-methyl-3-phenylpiperazine-2,5-dione (9) and the optically active 1-methyl-3-phenylpiperazine (11) are S-isomers.
<33> A method of producing optically active 1-methyl-3-phenylpiperazine-2,5-dione (9), comprising cyclizing the optically active compound (4) or salt thereof.
<34> The method according to <33>, wherein cyclization is carried out by at least one treatment selected from the group consisting of a heat treatment, pressure reduction treatment, base treatment and acid treatment.
<35> A method of producing an optically active compound (4) or salt thereof, comprising removing the protective group Z¹ from an optically active compound (3).
<36> A method of producing an optically active compound (3) or salt thereof, comprising reacting a sarcosine compound (1) or a reactive derivative at its carboxyl group with an optically active phenylglycine compound (2).
<37> A method of producing optically active 1-methyl-3-phenylpiperazine-2,5-dione (9), comprising cyclizing optically active compound (8) or salt thereof.
<38> The method according to <37>, wherein cyclization is carried out by at least one treatment selected from the group consisting of a heat treatment, pressure reduction treatment, base treatment and acid treatment.
<39> A method of producing an optically active compound (8) or salt thereof, comprising removing the protective group Z² from an optically active compound (7).
<40> A method of producing an optically active compound (7) or salt thereof, comprising reacting an optically active phenylglycine compound (5) or a reactive derivative at its carboxyl group with a sarcosine compound (6).
<41> A method of producing an optically active 1-(3-hydroxymethyl-2-pyridyl)-4-methyl-2-phenylpiperazine (14) or salt thereof, comprising reducing optically active 2-(4-methyl-2-phenylpiperazin-1-yl)-3-pyridinecarbaldehyde (13) or salt thereof with a reducing agent.
<42> The method according to <41>, wherein the reducing agent is sodium borohydride.
<43> A method of producing an optically active 2-(4-methyl-2-phenylpiperazin-1-yl)-3-pyridinecarbaldehyde (13) or salt thereof, comprising reducing optically active 1-(3-cyano-2-pyridyl)-4-methyl-2-phenylpiperazine (12) or salt thereof with a reducing agent.
<44> The method according to <43>, wherein the reducing agent is hydrogenated diisobutylaluminum.
<45> A method of producing optically active 1-(3-cyano-2-pyridyl)-4-methyl-2-phenylpiperazine (12) or salt thereof, comprising condensing optically active 1-methyl-3-phenylpiperazine (11) or salt thereof with 2-halogeno-3-cyanopyridine in the presence of a base.

### BEST MODES FOR CARRYING OUT THE INVENTION

The present invention will be illustrated in detail below.

Definitions of marks used in the present invention are as described below.

R¹ or R² represents an optionally substituted alkyl group having 1 to 6 carbon atoms, an optionally substituted cycloalkyl group having 3 to 7 carbon atoms or an optionally substituted aralkyl group having 7 to 12 carbon atoms.

"alkyl group having 1 to 6 carbon atoms" in "optionally substituted alkyl group having 1 to 6 carbon atoms" may be linear or branched, and examples thereof include a methyl group, ethyl group, propyl group, isopropyl group, butyl group, isobutyl group, sec-butyl group, tert-butyl group, pentyl group, isopentyl group, neopentyl group, hexyl group and the like. The alkyl group may have a substituent at a substitutable position, and as such a substituent, for example, C₁₋₃ alkoxy groups (e.g., methoxy group, ethoxy group, propoxy group, isopropoxy group, etc.) and the like are listed. The number of the substituent is not particularly restricted, and preferably 1 to 3, and the substituents may be the same or different.

Examples of "cycloalkyl group having 3 to 7 carbon atoms" in "optionally substituted cycloalkyl group having 3 to 7 carbon atoms" include a cyclopropyl group, cyclobutyl group, cyclopentyl group, cyclohexyl group, cycloheptyl group and the like. The cycloalkyl group may have a substituent at a substitutable position, and as such a substituent, for example, C₁₋₄ alkyl groups (e.g., methyl group, ethyl group, propyl group, isopropyl group, butyl group, etc.), C₁₋₄ alkoxy groups (e.g., methoxy group, ethoxy group, propoxy group, isopropoxy group, butoxyl group, etc.), C₆₋₁₀ aryl groups (e.g., phenyl group, naphthyl group, etc.) and the like are listed. The number of the substituent is not particularly restricted, and preferably 1 to 3, and the substituents may be the same or different.

"aralkyl group having 7 to 12 carbon atoms" in "optionally substituted aralkyl group having 7 to 12 carbon atoms" is an aralkyl group having a total carbon number of 7 to 12 carrying a phenyl group or naphthyl group substituted at any position of "alkyl group having 1 to 6 carbon atoms" defined above, and examples thereof include a benzyl group, 1- or 2-phenylethyl group, 1-, 2- or 3-phenylpropyl group, 1-, 2-, 3- or 4-phenylbutyl group, 1-, 2-, 3-, 4- or 5-phenylpentyl group, 1-, 2-, 3-, 4-, 5- or 6-phenylhexyl group, 1- or 2-naphthylmethyl group, 1- or 2-(1-naphthyl)ethyl group, 1- or 2-(2-naphthyl)ethyl group and the like. The aralkyl group may have a substituent at a substitutable position, and as such a substituent, for example, the same groups as the substituents which may be carried on the above-described alkyl group are mentioned. The number of the substituent is not particularly restricted, and preferably 1 to 3, and the substituents may be the same or different.

R¹ and R² represent preferably a methyl group, ethyl group, propyl group, isopropyl group, butyl group, benzyl group and the like. Here, when an amino group-protective group Z¹ or Z² is removed by catalytic reduction, as described below, a benzyl group is not preferable for preventing removal of R¹ or R².

Z¹ and Z² represent an amino group-protective group.

As "amino group-protective group", conventionally known amino group-protective groups generally used in peptide synthesis are mentioned, and preferable are protective groups which are easily removed and show little tendency of recemization in removal. Examples of such protective groups include optionally substituted benzyloxycarbonyl groups (as the substituent, for example, methyl group, methoxy group and the like are listed), tert-butoxycarbonyl group and the like, and of them, preferable are a benzyloxycarboxyl group and tert-butoxycarbonyl group.

A carbon atom with * represents an asymmetrical carbon atom, meaning that the ratio of the substituents of R configuration to the substituents of S configuration on the asymmetrical carbon atom is any value from 0:100 to 100:0. However, when the compound is "optically active compound", 50:50 is excluded.

The production method of the present invention will be described below.

In the present invention, optically active 1-methyl-3-phenylpiperazine (11) or salt thereof can be produced by the following method 1. 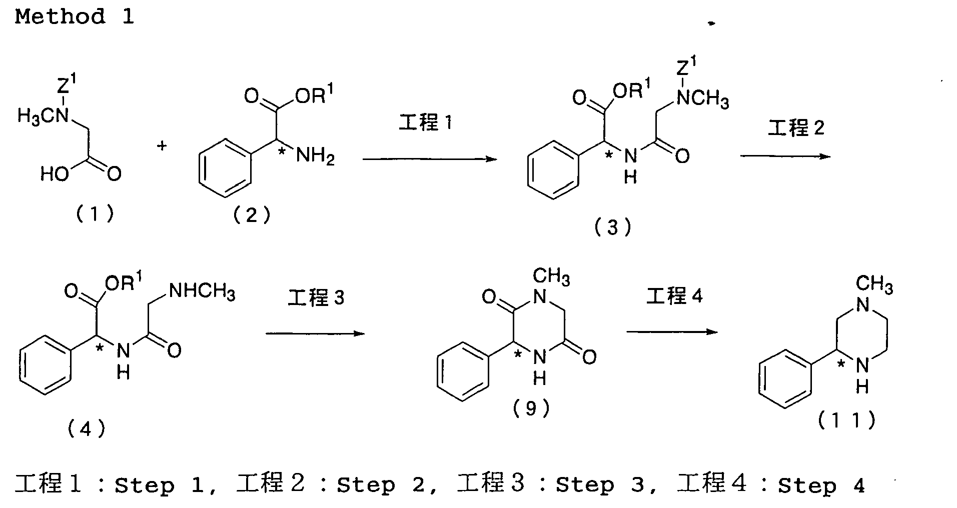 (in the formulae, marks are as defined above.).

Each step will be described below.

### (step 1)

A sarcosine compound (1) or a reactive derivative at its carboxyl group can be reacted with an optically active phenylglycine compound (2), to produce an optically active compound (3) or salt thereof.

Here, the phenylglycine compound (2) is optically active, and its enantiomeric excess takes any value of larger than 0% ee and up to 100% ee. Here, for obtaining the targeted optically active 1-methyl-3-phenylpiperazine or salt thereof with good optical purity, the enantiomeric excess of the above-described optically active phenylglycine compound (2) is preferably 95% ee or more, more preferably 99% or more, particularly preferably 100% ee.

The above-described reaction can be carried out by (i) a method of reacting a reactive derivative at a carboxyl group of a sarcosine compound (1) with an optically active phenylglycine compound (2), (ii) a method of reacting a sarcosine compound (1) with an optically active phenylglycine compound (2) in the presence of a condensation agent, and the like. These methods will be described below.

### Method of (i)

In this method, a reactive derivative at a carboxyl group of a sarcosine compound (1) is usually reacted with an optically active phenylglycine compound (2) in the presence of a base.

As the reactive derivative at a carboxyl group of a sarcosine compound (1), conventionally known derivatives can be used, and examples thereof include acid halides, acid anhydrides (symmetric acid anhydrides, mixed acid anhydrides), active esters, active amides, and the like.

The acid halide includes acid chloride, acid bromide and the like. These can be prepared by treating a sarcosine compound (1) with a halogenating agent such as SOCl₂, oxalyl chloride, SO₂Cl₂, PCl₃, PCl₅, POCl₃, PBr₃ and the like.

The mixed acid anhydride includes sarcosine compound (1)-dialkyl phosphate (for example, diC₁₋₆ alkyl phosphates) mixed acid anhydrides, sarcosine compound (1)-diphenyl phosphate mixed acid anhydrides, sarcosine compound (1)-dialkyl phosphite (for example, diC₁₋₆ alkyl phosphites) mixed acid anhydrides, sarcosine compound (1)-alkyl carbonate (for example, C₁₋₆ alkyl carbonates) mixed acid anhydrides, sarcosine compound (1)-aliphatic carboxlic acid (for example, pivalic acid, trichloroacetic acid and the like) mixed acid anhydrides, sarcosine compound (1)-sulfonic acid (methanesulfonic acid, p-toluenesulfonic acid and the like) mixed acid anhydrides, and the like.

The active ester includes a p-nitrophenyl ester of a sarcosine compound (1), an ester of a sarcosine compound (1) and N-hydroxysuccinimide, an ester of a sarcosine compound (1) and 1-hydroxybenzotriazole, and the like.

The active amide includes an amide of a sarcosine compound (1) and imidazole, an amide of a sarcosine compound (1) and dimethylimidazole, an amide of a sarcosine compound (1) and triazole, and the like. These can be prepared, for example, by reacting a sarcosine compound (1) with N,N'-carbonyldiimidazole (CDI) and the like.

A case in which the reactive derivative at a carboxyl group of a sarcosine compound (1) is a sarcosine compound (1)-alkyl carbonate mixed acid anhydride will be explained as an example.

The sarcosine compound (1)-alkyl carbonate mixed acid anhydride can be prepared by reacting a sarcosine compound (1) with an alkyl chloroformate in the presence of a base. When the sarcosine compound (1) is, for example, a suitable salt such as a triethylamine salt and the like, the reaction can be performed without particularly adding a base. The addition order of a sarcosine compound (1), alkyl chloroformate and base is not particularly restricted, and it is usual that a mixture of a sarcosine compound (1) and a base is added to an alkyl chloroformate, as described in JP-A No. 2002-53543.

Examples of the alkyl chloroformate include alkyl chloroformates having 2 to 7 carbon atoms such as methyl chloroformate, ethyl chloroformate, butyl chloroformate, isobutyl chloroformate and the like.

The amount of the alkyl chloroformate is usually 0.9 to 1.1 mol, preferably 0.95 to 1.05 mol based on a mol of a sarcosine compound (1), from the standpoints of yield and efficiency.

The base includes organic bases such as trialkylamines (for example, trimethylamine, triethylamine, tripropylamine, ethyldiisopropylamine, etc.), N-alkylmorpholines (for example, N-methylmorpholine, N-ethylmorpholine, etc.), and the like.

The amount of the base is usually 0.9 to 1.1 mol, preferably 0.95 to 1.05 mol based on 1 mol of a sarcosine compound (1), from the standpoints of yield and efficiency.

Preparation of a sarcosine compound (1)-alkyl carbonate mixed acid anhydride is usually carried out in the presence of a solvent. Examples of the solvent include ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like; ester solvents such as ethyl acetate, butyl acetate and the like; ketone solvents such as methyl isobutyl ketone and the like, and these can be used singly or in the form of a mixture of two or more solvents.

The amount of the solvent is usually 1 to 5 L, preferably 1.5 to 3 L based on 1 mol of a sarcosine compound (1), from the standpoints of stirring efficiency and reaction speed.

The reaction temperature is usually -30 to 25°C, preferably -20 to 10°C from the standpoints of industrial applicability, reaction speed, by-product prevention and the like. The reaction time is usually 5 minutes to 2 hours, preferably 10 minutes to 1 hour depending on the reaction temperature, use amounts of raw materials and the like.

When a sarcosine compound (1) is obtained in the form of salt, if the salt is undesirable for the reaction, it is advantageous to add an acid to previously liberate the salt.

By thus previously preparing a sarcosine compound (1)-alkyl carbonate mixed acid anhydride before reacting with an optically active phenylglycine compound (2), an optically active compound (3) can be produced. Usually, the above-described preparation of a sarcosine compound (1)-alkyl carbonate mixed acid anhydride and the subsequent reaction with an optically active phenylglycine compound (2) can be carried out in an identical vessel.

The addition order of a sarcosine compound (1)-alkyl carbonate mixed acid anhydride and an optically active phenylglycine compound (2) is not particularly restricted, and an optically active phenylglycine compound (2) may be added to a sarcosine compound (1)-alkyl carbonate mixed acid anhydride, or a sarcosine compound (1)-alkyl carbonate mixed acid anhydride may be added to an optically active phenylglycine compound (2).

The amount of an optically active phenylglycine compound (2) is usually 0.9 to 1.1 mol, preferably 0.95 to 1.05 mol based on 1 mol of a sarcosine compound (1).

This reaction is usually carried out in the presence of a solvent, and usually, the same solvents as those used for preparation of a sarcosine compound (1)-alkyl carbonate mixed acid anhydride are used. It is also possible to carry out the reaction in the presence of water.

The reaction temperature is usually -30 to 25°C, preferably -20 to 10°C from the standpoints of industrial applicability, reaction speed, by-product prevention and the like. The reaction time is usually 5 minutes to 2 hours, preferably 10 minutes to 1 hour depending on the reaction temperature, use amounts of raw materials and the like.

When an optically active phenylglycine compound (2) is obtained in the form of salt, if the salt is undesirable for the reaction, it is advantageous to add a base to previously liberate the salt.

### Method of (ii)

In this method, a sarcosine compound (1) and an optically active phenylglycine compound (2) are reacted in the presence of a condensation agent. The addition order of a sarcosine compound (1), optically active phenylglycine compound (2) and condensation agent is not particularly restricted, and usually, a sarcosine compound (1), optically active phenylglycine compound (2) and condensation agent are added into the reaction system in this order.

The condensation agent includes N,N'-dicyclohexylcarbodiimide (DCC), N-cyclohexyl-N'-morpholinoethylcarbodiimide, N,N'-diisopropylcarbodiimide and the like.

The amount of the condensation agent is usually 0.8 to 1.5 mol, preferably 0.9 to 1.3 mol based on 1 mol of a sarcosine compound (1), from the standpoints of yield and efficiency.

The amount of an optically active phenylglycine compound (2) is usually 0.9 to 1.1 mol, preferably 0.95 to 1.05 mol based on 1 mol of a sarcosine compound (1).

This reaction is carried out usually in the presence of a solvent, and examples of the solvent include ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like; ester solvents such as ethyl acetate, butyl acetate and the like; ketone solvents such as methyl isobutyl ketone and the like, and these can be used singly or in the form of a mixture of two or more solvents.

The amount of the solvent is usually 1 to 5 L, preferably 1.5 to 3 L based on 1 mol of a sarcosine compound (1), from the standpoints of stirring efficiency and reaction speed.

The reaction temperature is usually -30 to 25°C, preferably -20 to 0°C from the standpoints of industrial applicability, reaction speed, by-product prevention and the like. The reaction time is usually 5 minutes to 2 hours, preferably 10 minutes to 1 hour depending on the reaction temperature, use amounts of raw materials and the like.

When a sarcosine compound (1) and an optically active phenylglycine compound (2) are obtained in the form of salt, if the salt is undesirable for the reaction, it is advantageous to add an acid or base to previously liberate the salt.

As the sarcosine compound (1) used as a starting raw material, commercially available products may be used, alternatively, the sarcosine compound (1) can be produced by conventionally known methods using reagents for introducing sarcosine and a protective group Z¹.

As the optically active phenylglycine compound (2), commercially available products may be used, alternatively, the optically active phenylglycine compound (2) can be produced by reaction of optically active phenylglycine with R¹OH (for example, methanol, ethanol, benzyl alcohol and the like) by conventionally known methods.

Isolation of thus produced optically active compound (3) can be carried out by subjecting the reaction liquid to post treatments according to ordinary methods (for example, neutralization, extraction, washing with water, crystallization and the like). The optically active compound (3) can be purified by re-crystallization, extraction-purification, adsorption treatment with activated carbon, silica, alumina and the like, chromatography methods such as silica gel column chromatography and the like, and for example, the extraction solution itself may be subjected to the subsequent step without particular purification, or a residue after distillation off of a solvent may be subjected to the subsequent step without particular purification. (step 2)

In this step, a protective group Z¹ can be removed from an optically active compound (3), to produce an optically active compound (4).

The protective group Z¹ removal method is appropriately selected depending on the protective group, and conventionally known methods can be adopted. For example, when (i) the protective group Z¹ is an optionally substituted benzyloxycarbonyl group or the like, it is preferable to perform removal by catalytic reduction. When (ii) the protective group Z¹ is a tert-butoxycarbonyl group or the like, it is preferable to perform removal by acid treatment.

Catalytic reduction in (i) is usually carried out by blowing hydrogen in the presence of a reduction catalyst, or by applying hydrogen pressure in an autoclave. The reduction catalyst includes palladium carbon, hydrogenated palladium carbon, palladium chloride, palladium black and the like, and preferable is palladium carbon from the standpoints of economy and reagent safety.

The amount of the reducing catalyst is, in terms of metal, usually 2 to 30 g, preferably 5 to 15 g based on 1 mol of an optically active compound (3), from the standpoints of reaction completion and economy.

Catalytic reduction is carried out usually in the presence of a solvent, and examples of the solvent include acid solvents such as acetic acid, formic acid and the like; ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like; alcohol solvents such as methanol, ethanol, propanol, isopropanol and the like; ester solvents such as methyl acetate, ethyl acetate, propyl acetate and the like, and these can be used singly or in the form of a mixture of two or more solvents.

The amount of the solvent is not particularly restricted, and usually 2 to 8 L, preferably 3 to 5 L based on 1 mol of an optically active compound (3).

The catalytic reduction temperature is usually 16 to 50°C, preferably 20 to 35°C from the standpoints of industrial applicability, reaction speed, by-product prevention and the like. The reduction time is usually 0.5 to 5 hours, preferably 1 to 3 hours depending on the reduction temperature, use amount of a catalyst and the like.

When a protective group Z¹ is removed by-catalytic reduction, if R¹ in an optically active compound (3) is also removed simultaneously (for example, in the case of a benzyl group), cyclization in the subsequent step 3 is difficult, thus, it is preferable to select groups which are not removed by catalytic reduction such as, for example, a methyl group, ethyl group, propyl group, isopropyl group, butyl group and the like.

In the case of acid treatment in (ii), mentioned as the acid are trifluoroacetic acid, hydrobromic acid/acetic acid, hydrogen chloride/methanol, and the like.

The amount of the acid is usually 5 to 50 mol, preferably 10 to 30 mol based on 1 mol of an optically active compound (3), from the standpoints of reaction completion, yield, efficiency and the like.

In the acid treatment, the above-described acids may be used as a solvent simultaneously, alternatively, a solvent may be used separately. Examples of the solvent include acid solvents such as acetic acid and the like; ether solvents such as tetrahydrofuran, dimethoxyethane, diethoxyethane, dioxane, cyclopentyl methyl ether and the like; hydrocarbon solvents such as toluene, xylene, heptanes, hexane and the like; alcohol solvents such as methanol, ethanol, propanol, isopropanol, butanol and the like; ester solvents such as ethyl acetate, butyl acetate and the like; ketone solvents such as methyl isobutyl ketone and the like, and these can be used singly or in the form of a mixture of two or more solvents.

The amount of the solvent is not particularly restricted, and usually 1 to 5 L, preferably 1.5 to 3 L based on 1 mol of an optically active compound (3).

The acid treatment temperature is usually -20 to 40°C, preferably 0 to 30°C from the standpoints of industrial applicability, reaction speed, by-product prevention and the like. The reduction time is usually 0.5 to 5 hours, preferably 1 to 3 hours depending on treatment temperature, use amount of an acid and the like.

Isolation of thus produced optically active compound (4) can be carried out by subjecting the reaction liquid to post treatments according to ordinary methods (for example, neutralization, extraction, washing with water, crystallization, salt formation with inorganic acid or organic acid, and the like). The optically active compound (4) can be purified by re-crystallization, extraction-purification, adsorption treatment with activated carbon, silica, alumina and the like, chromatography methods such as silica gel column chromatography and the like, and for example, the extraction solution itself may be subjected to the subsequent step without particular purification, or a residue after distillation off of a solvent may be subjected to the subsequent step without particular purification.

### (step 3)

In this step, an optically active compound (4) can be cyclized, to produce an optically active 1-methyl-3-phenylpiperazine-2,5-dione (9).

The cyclization method includes (i) method by heat treatment, (ii) method by pressure reduction treatment, (iii) method by base treatment, (iv) method by acid treatment, or a combination of them, and the like.

The treatment temperature in the (i) method by heat treatment is usually 20 to 100°C, preferably 25 to 80°C, from the standpoints of reaction speed and prevention of by-product generation. The treatment time is usually 1 hour to 4 days, preferably 2 hours to 1 day depending on heating temperature and the like. This method can be carried out without solvent.

The pressure in the (ii) method by pressure reduction treatment is usually 0.13 to 13.3 kPa, preferably 0.7 to 6.7 kPa, from the standpoints of industrial applicability and reaction speed. The treatment time is usually 1 hour to 4 days, preferably 2 hours to 1 day depending on the degree of pressure reduction. This method can be carried out without solvent.

In the (iii) method by base treatment, the treatment is carried out using an organic base (for example, triethylamine and the like), sodium bicarbonate aqueous solution, sodium carbonate aqueous solution, potassium carbonate aqueous solution, and the like.

The use amount of the base is not particularly restricted, and amounts causing liberation of an optically active compound (4) may be permissible.

In this method, the above-described bases may be used as a solvent simultaneously, alternatively, a solvent may be used separately. Examples of the solvent include ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like; alcohol solvents such as methanol, ethanol, propanol, isopropanol, butanol and the like; ester solvents such as ethyl acetate, butyl acetate and the like; ketone solvents such as methyl isobutyl ketone and the like, and these can be used singly or in the form of a mixture of two or more solvents.

The amount of the solvent is not particularly restricted, and usually 0.5 to 5 L, preferably 1 to 3 L based on 1 mol of an optically active compound (4).

The treatment temperature is usually 20 to 100°C, preferably 25 to 80°C from the standpoints of reaction speed and prevention of by-product. The treatment time is usually 1 hour to 4 days, preferably 2 hours to 1 day depending on treatment temperature, use amount of a base, and the like.

In the (iv) method by acid treatment, the treatment is carried out using polyphosphoric acid, sulfuric acid, hydrochloric acid, phosphoric acid, p-toluenesulfonic acid, zinc chloride, aluminum chloride and the like, and of them, polyphosphoric acid, sulfuric acid, phosphoric acid, zinc chloride and the like are suitably used from the standpoint of reaction selectivity.

The amount of the acid is usually 0.1 to 1 mol, preferably 0.2 to 0.5 mol based on 1 mol of an optically active compound (4), from the standpoints of yield and efficiency.

In this method, the above-described acids may be used as a solvent simultaneously, alternatively, a solvent may be used separately. Examples of the solvent include acid solvents such as acetic acid and the like; ether solvents such as tetrahydrofuran, dimethoxyethane, diethoxyethane, dioxane, cyclopentyl methyl ether and the like; hydrocarbon solvents such as toluene, xylene, heptanes, hexane and the like; alcohol solvents such as methanol, ethanol, propanol, isopropanol, butanol and the like; ester solvents such as ethyl acetate, butyl acetate and the like; ketone solvents such as methyl isobutyl ketone and the like, and these can be used singly or in the form of a mixture of two or more solvents.

The amount of the solvent is not particularly restricted, and usually 2 to 20 L, preferably 4 to 8 L based on 1 mol of an optically active compound (4).

The treatment temperature is usually 0 to 50°C, preferably 10 to 30°C from the standpoints of reaction speed and by-product prevention. The treatment time is usually 0.5 to 5 hours, preferably 1 to 2 hours depending on treatment temperature, use amount of an acid and the like.

It is also possible to carry out the reaction of the step 3 without any other procedures following the step 2, depending on the kind of an optically active compound (4), the above-described reaction conditions of the step 2, post treatment conditions and the like.

Isolation of thus produced optically active 1-methyl-3-phenylpiperazine-2,5-dione (9) can be carried out by subjecting the reaction liquid to post treatments according to ordinary methods (for example, neutralization, extraction, washing with water, crystallization and the like). The optically active 1-methyl-3-phenylpiperazine-2,5-dione (9) can be purified by re-crystallization, extraction-purification, adsorption treatment with activated carbon, silica, alumina and the like, chromatography methods such as silica gel column chromatography and the like, and for example, the extraction solution itself may be subjected to the subsequent step without particular purification, or a residue after distillation off of a solvent may be subjected to the subsequent step without particular purification. (step 4)

In this step, an optically active 1-methyl-3-phenylpiperazine-2,5-dione (9) can be reduced to produce optically active 1-methyl-3-phenylpiperazine (11) or salt thereof.

Reduction is preferably carried out using a reducing agent. The addition order of optically active 1-methyl-3-phenylpiperazine-2,5-dione (9) and reducing agent is not particularly restricted, and usually, optically active 1-methyl-3-phenylpiperazine-2,5-dione (9) is added to a suspension of a reducing agent.

The reducing agent includes lithium aluminum hydride, diisobutylaluminum hydride, lithium borohydride, sodium borohydride, borane-tetrahydrofuran complex, borane-diemthyl sulfide complex, and the like.

The amount of the reducing agent is usually 5 to 50 g equivalent, preferably 10 to 25 g equivalent, in terms of the amount of active hydrogen in the reducing agent, based on 1 mol of optically active 1-methyl-3-phenylpiperazine-2,5-dione (9) or salt thereof from the standpoints of yield and efficiency.

Reduction is carried out usually in the presence of a solvent, and examples of the solvent include ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like, and these can be used singly or in the form of a mixture of two or more solvents.

The amount of the solvent is usually 1 to 10 L, preferably 2 to 7 L based on 1 mol of optically active 1-methyl-3-phenylpiperazine-2,5-dione (9) or salt thereof, from the standpoints of stirring efficiency and reaction speed.

The reduction temperature is usually 0 to 100°C, preferably 25 to 70°C from the standpoints of reaction speed and by-product prevention. The reduction time is usually 0.5 to 10 hours, preferably 1 to 5 hours depending on reduction temperature, amount of active hydrogen in the reducing agent, and the like.

Isolation of thus produced optically active 1-methyl-3-phenylpiperazine (11) can be carried out by subjecting the reaction liquid to post treatments according to ordinary methods (for example, filtration, neutralization, extraction, washing with water, crystallization, distillation and the like). The optically active 1-methyl-3-phenylpiperazine (11) can be purified by re-crystallization, extraction-purification, adsorption treatment with activated carbon, silica, alumina and the like, chromatography methods such as silica gel column chromatography and the like. Further, it can be purified by crystallizing as a salt.

In the above-described reactions of the steps 1 to 4, steric configurations of raw materials and targeted product are approximately kept. That is, when an optically active phenylglycine compound (2) in the form of S-isomer (L-isomer) is used, (S)-1-methyl-3-phenylpiperazine (11) can be produced, and when a R-isomer (D-isomer) is used, (R)-1-methyl-3-phenylpiperazine (11) can be produced, and those having an enantiomeric excess of preferably 95% ee or more, more preferably 99% ee or more can be produced.

Optically active 1-methyl-3-phenylpiperazine (11) or salt thereof can also be produced by the following method 2. 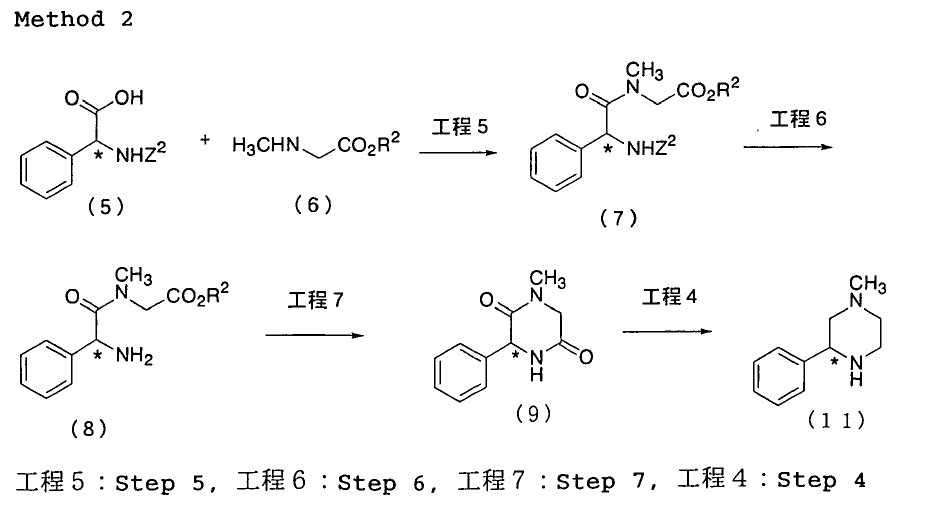 (in the formulae, marks are as defined above.)

Each step will be described below.

### (Step 5)

In this step, an optically active phenylglycine compound (5) can be reacted with a sarcosine compound (6) to product an optically active compound (7).

Here, the phenylglycine compound (5) is optically active, and its enantiomeric excess takes any value of larger than 0% ee and up to 100% ee. Here, for obtaining the targeted optically active 1-methyl-3-phenylpiperazine or salt thereof with good optical purity, the enantiomeric excess of the above-described optically active phenylglycine compound (5) is preferably 95% ee or more, more preferably 99% or more, particularly preferably 100% ee.

The above-described reaction can be carried out by (i) a method of reacting a reactive derivative at a carboxyl group of an optically active phenylglycine compound (5) with a sarcosine compound (6), (ii) a method of reacting an optically active phenylglycine compound (5) with a sarcosine compound (6) in the presence of a condensation agent, and the like. This step can be carried out in the same manner as in the step 1, using an optically active phenylglycine compound (5) instead of a sarcosine compound (1), and using a sarcosine compound (6) instead of an optically active phenylglycine compound (2) in the step 1 of the above-described method 1.

### (Step 6)

In this step, a protective group Z² can be removed from an optically active compound (7) to produce an optically active compound (8). This step can be carried out in the same manner as in the step 2 in the above-described method 1.

### (Step 7)

In this step, an optically active compound (8) can be cyclized to produce an optically active 1-methyl-3-phenylpiperazine-2,5-dione (9). This step can be carried out in the same manner as in the step 3 in the above-described method 1.

### (Step 4)

This step is the same as the step 4 in the above-described method 1.

In the above-described steps 5 to 7 and step 4, steric configurations of raw materials and targeted product are approximately kept. That is, when an optically active phenylglycine compound (5) in the form of S-isomer (L-isomer) is used, (S)-1-methyl-3-phenylpiperazine (11) can be produced, and when a R-isomer (D-isomer) is used, (R)-1-methyl-3-phenylpiperazine (11) can be produced, and those having an enantiomeric excess of preferably 95% ee or more, more preferably 99% ee or more can be produced.

The present inventors have produced optically active 1-(3-hydroxymethyl-2-pyridyl)-4-methyl-2-phenylpiperazine (14) or salt thereof according to the same method as described in US 4062848 and US 6852855 (method for racemate) from thus produced optically active 1-methyl-3-phenylpiperazine (11) or salt thereof, further, tried to produce optically active mirtazapine or salt thereof according to the same method as described in WO 2005/005410. Its route is shown below. 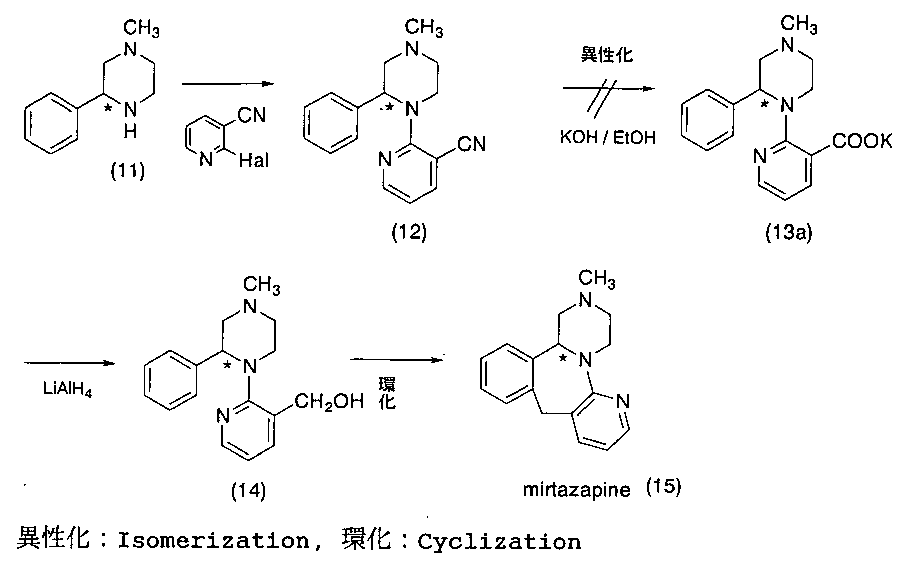 (in the formulae, Hal represents a halogen atom (preferably, chlorine atom, bromine atom)).

However, it has been clarified that, in the above-described route, though optically active 1-(3-cyano-2-pyridyl)-4-methyl-2-phenylpiperazine (12) or salt thereof (hereinafter, these are summarized as optically active 1-(3-cyano-2-pyridyl)-4-methyl-2-phenylpiperazine (12) in some cases) is obtained from optically active 1-methyl-3-phenylpiperazine (11) or salt thereof (hereinafter, these are summarized as optically active 1-methyl-3-phenylpiperazine (11) in some cases) while retaining its steric configuration, in the next step, its steric configuration is not retained, and the resultant 2-(4-methyl-2-phenylpiperazin-1-yl)-3-pyridine carboxylic acid (13a) or salt thereof is isomerized.

Then, the present inventors have investigated a route progressing not via this 2-(4-methyl-2-phenylpiperazin-1-yl)-3-pyridine carboxylic acid or salt thereof (13a), and found that, if a route is used via optically active 2-(4-methyl-2-phenylpiperazin-1-yl)-3-pyridinecarbaldehyde (13) or salt thereof (hereinafter, these are summarized as optically active 2-(4-methyl-2-phenylpiperazin-1-yl)-3-pyridinecarbaldehyde (13) in some cases), optically active 1-(3-hydroxymethyl-2-pyridyl)-4-methyl-2-phenylpiperazine (14) or salt thereof (hereinafter, these are summarized as optically active 1-(3-hydroxymethyl-2-pyridyl)-4-methyl-2-phenylpiperazine (14) in some cases) can be produced while retaining its steric configuration (steps 11 to 13), further, optically active mirtazapine (15) or salt thereof (hereinafter, these are summarized as optically active mirtazapine (15) in some cases) can be produced while retaining its steric configuration (step 14).

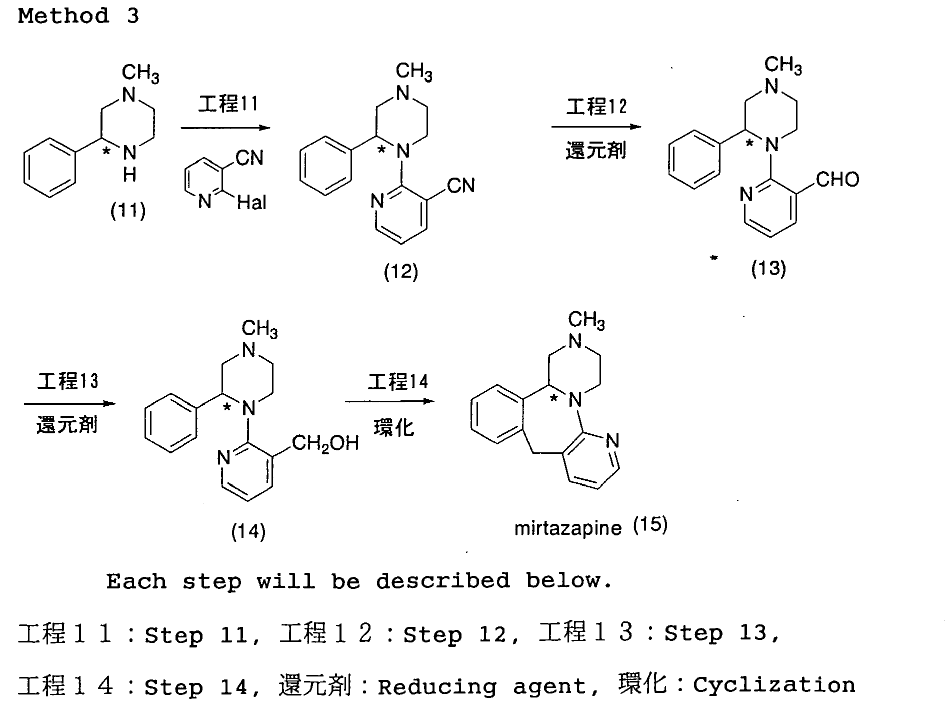

### Step 11

In this step, optically active 1-methyl-3-phenylpiperazine (11) can be condensed with 2-halogeno-3-cyanopyridine in the presence of a base to produce optically active 1-(3-cyano-2-pyridyl)-4-methyl-2-phenylpiperazine (12).

Preferable as the 2-halogeno-3-cyano-pyridine are 2-chloro-3-cyano-pyridine and 2-bromo-3-cyano-pyridine.

The amount of the 2-halogeno-3-cyano-pyridine is usually 1 to 2 mol, preferably 1.3 to 1.6 mol based on 1 mol of optically active 1-methyl-3-phenylpiperazine (11) from the standpoints of yield and efficiency.

The base includes organic bases such as triethylamine, trimethylamine, tributylamine and the like.

The amount of the base is usually 1 to 2 mol, preferably 1.2 to 1.6 mol based on 1 mol of optically active 1-methyl-3-phenylpiperazine (11) from the standpoints of yield and efficiency.

The reaction is carried out usually in the presence of a solvent, and examples of the solvent include dimethylformamide (DMF), dimethylacetamide (DMAC), 1,3-dimethyl-2-imidazolidinone (DMI), dimethyl sulfoxide (DMSO) and the like, and these can be used singly or in the form of a mixture of two or more solvents. Among others, DMF is preferable from the standpoints of reaction time and economy.

The amount of the solvent is usually 0.1 to 2 L, preferably 0.2 to 1 L based on 1 mol of optically active 1-methyl-3-phenylpiperazine (11), from the standpoints of stirring efficiency and reaction speed.

The reaction temperature is usually 100 to 150°C, preferably 120 to 140°C from the standpoints of reaction speed and by-product prevention. The reaction time is usually 5 to 50 hours, preferably 8 to 20 hours depending on reaction temperature, use amounts of raw materials, and the like.

When optically active 1-methyl-3-phenylpiperazine (11) is obtained in the form of salt, if the salt is undesirable for the reaction, it is advantageous to add a base to previously liberate the salt.

Isolation of thus produced optically active 1-(3-cyano-2-pyridyl)-4-methyl-2-phenylpiperazine (12) can be carried out by subjecting the reaction liquid to post treatments according to ordinary methods (for example, filtration, neutralization, extraction, washing with water, crystallization, distillation and the like). The optically active 1-(3-cyano-2-pyridyl)-4-methyl-2-phenylpiperazine (12) can be purified by re-crystallization, extraction-purification, adsorption treatment with activated carbon, silica, alumina and the like, chromatography methods such as silica gel column chromatography and the like. Further, it can also be obtained in the form of a salt with an acid such as oxalic acid, and for example, the extraction solution itself may be subjected to the subsequent step without particular purification, or a residue after distillation off of a solvent may be subjected to the subsequent step without particular purification.

### Step 12

In this step, optically active 1-(3-cyano-2-pyridyl)-4-methyl-2-phenylpiperazine (12) can be reduced with a reducing agent to produce optically active 2-(4-methyl-2-phenylpiperazin-1-yl)-3-pyridinecarbaldehyde (13). The addition order of optically active 1-(3-cyano-2-pyridyl)-4-methyl-2-phenylpiperazine (12) and reducing agent is not particularly restricted, and usually, a reducing agent is added to a solution of optically active 1-(3-cyano-2-pyridyl)-4-methyl-2-phenylpiperazine (12).

The reducing agent is not particularly restricted providing it can reduce a cyano group into a formyl group, and includes hydrogenated diisobutylaluminum, hydrogenated diisopropylaluminum, hydrogenated dihexylaluminum and the like. Of them, hydrogenated diisobutylaluminum is preferable from the standpoint of easy availability. The reducing agent is preferably dissolved previously in an organic solvent (preferably, toluene) in use, and in particular, a toluene solution of hydrogenated diisobutylaluminum is suitably used.

The amount of the reducing agent is usually 1.7 to 3.0 mol, preferably 2.0 to 2.5 mol based on 1 mol of optically active 1-(3-cyano-2-pyridyl)-4-methyl-2-phenylpiperazine (12), from the standpoints of yield and efficiency.

Reduction is carried out usually in the presence of a solvent, and examples of the solvent include ether solvents such as tetrahydrofuran, dimethoxyethane, diethoxyethane, dioxane, tert-butyl methyl ether, cyclopentyl methyl ether and the like; hydrocarbon solvents such as toluene, xylene, heptanes, hexane and the like, and these can be used singly or in the form of a mixture of two or more solvents. Of them, toluene is preferable from the standpoints of reaction time and economy.

The amount of the solvent is usually 500 to 2000 parts by weight, preferably 1000 to 1500 parts by weight based on 100 parts by weight of optically active 1-(3-cyano-2-pyridyl)-4-methyl-2-phenylpiperazine (12), from the standpoints of stirring efficiency and reaction speed.

Reduction is preferably carried out, for example, under an atmosphere of an inert gas such as a nitrogen gas, argon gas and the like.

The preferable reduction temperature is usually -70 to - 10°C. The reduction time is usually 0.5 to 12 hours, preferably 1 to 3 hours depending on the reduction temperature, use of a reducing agent, and the like.

When optically active 1-(3-cyano-2-pyridyl)-4-methyl-2-phenylpiperazine (12) is obtained in the form of salt, if the salt is undesirable for the reaction, it is advantageous to add a base to previously liberate the salt.

Reduction of optically active 1-(3-cyano-2-pyridyl)-4-methyl-2-phenylpiperazine (12) can be stopped by, for example, adding a lower alcohol to its reaction solution. As the lower alcohol, monovalent alcohols having 1 to 4 carbon atoms such as ethanol and the like can be suitably used. The amount of the lower alcohol is usually about 2 to 10 mol based on 1 mol of optically active 1-(3-cyano-2-pyridyl)-4-methyl-2-phenylpiperazine (12).

For decomposing a reducing agent, after completion of the reduction reaction, and for hydrolyzing the produced imide body into a carbaldehyde body, it is preferable to mix the resultant reaction mixture with water. The amount of water is usually about 200 to 1000 parts by weight based on 100 parts by weight of optically active 1-(3-cyano-2-pyridyl)-4-methyl-2-phenylpiperazine (12).

Thereafter, an alkali aqueous solution can be added to the reaction mixture, for dissolving and removing a reducing agent remaining in the reaction mixture, if necessary. Examples of such an alkali aqueous solution includes aqueous solutions of alkali metal hydroxides such as a sodium hydroxide aqueous solution, potassium hydroxide aqueous solution and the like.

Next, an aqueous layer is separated from the reaction mixture, a mineral acid such as sulfuric acid and the like is added to an organic layer, and the organic layer is heated at about 50°C for about 30 minutes to 1 hour, then, an alkali aqueous solution is added to render the layer alkaline, the liquid is partitioned, and dried and an organic solvent is removed according to ordinary methods.

Isolation of thus produced optically active 2-(4-methyl-2-phenylpiperazin-1-yl)-3-pyridinecarbaldehyde (13) can be carried out by subjecting the reaction liquid to post treatments according to ordinary methods (for example, filtration, neutralization, extraction, washing with water, crystallization, distillation and the like). The optically active 2-(4-methyl-2-phenylpiperazin-1-yl)-3-pyridinecarbaldehyde (13) can be purified by re-crystallization, extraction-purification, adsorption treatment with activated carbon, silica, alumina and the like, chromatography methods such as silica gel column chromatography and the like, and for example, the extraction solution itself may be subjected to the subsequent step without particular purification, or a residue after distillation off of a solvent may be subjected to the subsequent step without particular purification. Optically active 2-(4-methyl-2-phenylpiperazin-1-yl)-3-pyridinecarbaldehyde (13) can be obtained, for example, in the form of a salt such as an oxalate, hydrochloride, methane sulfonate and the like, and these salts can be obtained by usual methods.

### Step 13

In this step, optically active 2-(4-methyl-2-phenylpiperazin-1-yl)-3-pyridinecarbaldehyde (13) can be reduced with a reducing agent to produce optically active 1-(3-hydroxymethyl-2-pyridyl)-4-methyl-2-phenylpiperazine (14). The addition order of optically active 2-(4-methyl-2-phenylpiperazin-1-yl)-3-pyridinecarbaldehyde (13) and reducing agent is not particularly restricted, and usually, a reducing agent is added (preferably, added divisionally) to a solution of optically active 2-(4-methyl-2-phenylpiperazin-1-yl)-3-pyridinecarbaldehyde (13).

The reducing agent is not particularly restricted providing it can reduce a formyl group into a hydroxymethyl group, and includes sodium borohydride, sodium cyanoborohydride, lithium borohydride, and the like. Of them, sodium borohydride is preferable from the standpoints of easy availability and economy.

The amount of the reducing agent is usually 1.5 to 3 mol, preferably 2 to 2.5 mol based on 1 mol of optically active 2-(4-methyl-2-phenylpiperazin-1-yl)-3-pyridinecarbaldehyde (13), from the standpoints of yield and efficiency.

Reduction is carried out usually in the presence of a solvent, and examples of the solvent include lower alcohols such as methanol, ethanol and the like, hydrated solvents of them; solvent systems prepared by using a two-layer solvent composed of water and a hydrophobic organic solvent such as ethyl acetate and the like and allowing a phase transition catalyst such as tetrabutylammonium bromide and the like to present in this solvent; and the like. Of them, methanol is preferable.

The amount of the solvent is usually 400 to 2000 parts by weight, preferably 500 to 1000 parts by weight based on 100 parts by weight of optically active 2-(4-methyl-2-phenylpiperazin-1-yl)-3-pyridinecarbaldehyde (13), from the standpoints of stirring efficiency and reaction speed.

The reduction temperature is usually -10 to 50°C, preferably 0 to 10°C from the standpoints of reaction speed and by-product prevention. The reduction time is usually 0.5 to 5 hours, preferably 1 to 2 hours depending on reduction temperature, amount of a reducing agent, and the like.

When optically active 2-(4-methyl-2-phenylpiperazin-1-yl)-3-pyridinecarbaldehyde (13) is obtained in the form of salt, if the salt is undesirable for the reaction, it is advantageous to add a base to previously liberate the salt.

Isolation of thus produced optically active 1-(3-hydroxymethyl-2-pyridyl)-4-methyl-2-phenylpiperazine (14) can be carried out by subjecting the reaction liquid to post treatments according to ordinary methods (for example, filtration, neutralization, extraction, washing with water, crystallization, distillation and the like). The optically active 1-(3-hydroxymethyl-2-pyridyl)-4-methyl-2-phenylpiperazine (14) can be purified by re-crystallization, extraction-purification, adsorption treatment with activated carbon, silica, alumina and the like, chromatography methods such as silica gel column chromatography and the like, and for example, the extraction solution itself may be subjected to the subsequent step without particular purification, or a residue after distillation off of a solvent may be subjected to the subsequent step without particular purification.

### Step 14

In this step, optically active 1-(3-hydroxymethyl-2-pyridyl)-4-methyl-2-phenylpiperazine (14) can be cyclized to produce optically active mirtazapine (15).

Cyclization can be carried out in the same manner as described, for example, in WO 2005/005410, and for example, methods by treatment with an acid such as polyphosphoric acid, phosphoric acid, sulfuric acid, zinc chloride and the like are mentioned.

In the above-described reactions in the steps 11 to 14, steric configurations of optically active compounds are approximately kept. That is, when optically active 1-methyl-3-phenylpiperazine (11) in the form of S-isomer (L-isomer) is used, (S)-mirtazapine (15) can be produced, and when a R-isomer (D-isomer) is used, (R)-mirtazapine (15) can be produced, and those having an enantiomeric excess of preferably 95% ee or more, more preferably 99% ee or more can be produced.

In each step of the above-described methods 1 to 3, the sarcosine compound (1), optically active phenylglycine compound (2), optically active compound (4), optically active phenylglycine compound (5), sarcosine compound (6), optically active compound (8), optically active 1-methyl-3-phenylpiperazine (11), optically active 1-(3-cyano-2-pyridyl)-4-methyl-2-phenylpiperazine (12), optically active 2-(4-methyl-2-phenylpiperazin-1-yl)-3-pyridinecarbaldehyde (13), optically active 1-(3-hydroxymethyl-2-pyridyl)-4-methyl-2-phenylpiperazine (14) and optically active mirtazapine (15) may form salts. Examples of the salt include salts with alkali metals (for example, sodium, potassium, etc.); alkaline earth metals (for example, calcium, magnesium, etc.); organic bases (for example, trimethylamine, triethylamine, pyridine, picoline, dicyclohexylamine, etc.) and the like, and acid added salts of inorganic acids (for example, hydrochloric acid, hydrobromic acid, nitric acid, sulfuric acid, phosphoric acid, etc.); organic acids (for example, formic acid, acetic acid, oxalic acid, malonic acid, citric acid, fumaric acid, lactic acid, malic acid, succinic acid, tartaric acid, trifluoroacetic acid, etc.) and the like.

The optically active compound (3), optically active compound (4), optically active compound (7), optically active compound (8), 1-methyl-3-phenylpiperazine-2,5-dione (9'), (S)-1-methyl-3-phenylpiperazine (11) or salt thereof, (R)-1-methyl-3-phenylpiperazine (11) or salt thereof, (S)-1-(3-cyano-2-pyridyl)-4-methyl-2-phenylpiperazine (12) or salt thereof, (R)-1-(3-cyano-2-pyridyl)-4-methyl-2-phenylpiperazine (12) or salt thereof, (S)-2-(4-methyl-2-phenylpiperazin-1-yl)-3-pyridinecarbaldehyde (13) or salt thereof and (R)-2-(4-methyl-2-phenylpiperazin-1-yl)-3-pyridinecarbaldehyde (13) or salt thereof are novel compounds. Racemic bodies, optically active bodies and any mixtures of 1-methyl-3-phenylpiperazine-2,5-dione (9'), optically active compound (3), optically active compound (4), optically active compound (7) and optically active compound (8), of them, are also novel compounds, and any of them are included in the present invention.

The following examples will specifically illustrate the present invention, but the present invention is not limited to these examples at all. Abbreviation Sar represents sarcosine, Phg represents (S)-α-phenylglycine, and Z represents benzyloxycarbonyl group, respectively. In the following descriptions, (S)-α-phenylglycine is abbreviated as (S)-phenylglycine.

### <Synthesis of (S)-1-methyl-3-phenylpiperazine, Examples 1 to 4>

### Preparation of starting raw materials

According to an ordinary method, N-benzyloxycarbonylsarcosine can be produced from sarcosine and benzyloxycarbonyl chloride, and (S)-phenylglycine methyl ester hydrochloride can be produced from (S)-phenylglycine, methanol and thionyl chloride though commercially available products may also be used.

### Example 1

### (S)-N-[N-benzyloxycarbonylsarcosyl]phenylglycine methyl ester (Z-Sar-Phg-OMe)

N-benzyloxycarbonylsarcosine (2.23 g, 0.01 mol) was dissolved in tetrahydrofuran (THF, 10 ml), and triethylamine (1.39 ml, 0.01 mol) was added at -10°C or lower. To a solution of THF (10 ml) in ethyl chloroformate (0.956 ml, 0.01 mol) was added the above-described solution at -10°C or lower, the mixture was stirred at the same temperature for 10 minutes to obtain a suspension. Separately, (S)-phenylglycine methyl ester hydrochloride (2.22 g, 0.011 mol) ground in a mortar was suspended in THF (10 ml), triethylamine (1.53 ml, 0.011 mol) was added at -10°C or lower, and to this was added the above-described suspension at -10°C or lower, and the mixture was stirred for 15 minutes, further, stirred at 0°C for 30 minutes. Saturated sodium bicarbonate water (20 ml) was poured into this reaction mixture, and THF was once distilled off under reduced pressure. Extraction with ethyl acetate was performed (20 mlx2), the organic layer was washed sequentially with 10% hydrochloric acid (15 ml) and saturated sodium bicarbonate water (15 ml), dried over anhydrous magnesium sulfate, then, filtrated, and the solvent was distilled off under reduced pressure. The residue was purified by silica gel column chromatography (developing solvent: hexane:ethyl acetate = 4:3) to obtain 3.05 g(yield 82.4%) of a crystal of the title compound.

m.p. 118 to 119°C

¹H-NMR (ppm, CDCl₃): δ 3.02 (s, 3H, NMe), 3.72 (s, 3H, CO₂Me), 3.92 and 4.03 (2d, 2H, J=16.6 Hz, CH₂), 5.16 (brs, 2H, CH₂), 5.56 (d, 1H, J=7.3 Hz, CH), 7.20-7.40 (m, 10H, aromatic).

### Example 2

### (S)-N-sarcosyl-phenylglycine methyl ester (H-Sar-Phg-OMe)

A crystal (3.0 g) of (S)-N-[N-benzyloxycarbonylsarcosyl]phenylglycine methyl ester was dissolved in acetic acid (30 ml), 5% Pd-C (50% wet product, 0.3 g) was added to cause suspension, and the suspension was stirred in an autoclave under a hydrogen pressure of 5 kg/cm². After 40 minutes, Pd-C was removed by filtration, and most of acetic acid was distilled off under reduced pressure, to obtain an oil of the title compound.

¹H-NMR (ppm, CDCl₃): δ 2.52 (s, 3H, NMe), 3.48 (s, 2H, CH₂), 3.73 (s, 3H, CO₂Me), 5.60 (d, 1H, J=7.7 Hz, CH), 7.30-7.40 (m, 5H, aromatic).

### Example 3

### (S)-1-methyl-3-phenylpiperazine-2,5-dione

The oil obtained in Example 2 was allowed to stand at room temperature (25 °C) for 4 days, then, the deposited crystal was suspended with ethyl acetate (3 ml) and tert-butyl methyl ether (7 ml), then, the suspension was filtrated to obtain 1.07 g (yield 65.0%) of the title compound.

m.p. 185 to 190°C (sintered)

¹H-NMR (ppm, CDCl₃): δ 2.96 (s, 3H, NMe), 3.93 and 4.12 (2d, 2H, J=17.6 Hz, CH₂), 5.10 (d, 1H, J=2.9 Hz, CH), 6.67 (brs, 1H, NH), 7.30-7.43 (m, 5H, aromatic).

### Example 4

### (S)-1-methyl-3-phenylpiperazine

Under a nitrogen atmosphere, to a suspension of lithium aluminum hydride (0.46 g, 12.1 mmol) in THF (15 ml) was added (S)-1-methyl-3-phenylpiperazine-2,5-dione (0.5 g, 2.45 mmol), then, the mixture was refluxed for 1.5 hours. The mixture was ice-cooled, and ethyl acetate (5 ml), methanol (5 ml) and water (10 ml) were added, and the solvent was once distilled off under reduced pressure. Toluene (20 ml) and 10% NaOH aqueous solution (10 ml) were added, insoluble substances were removed by filtration through cerite, and a toluene layer was separated. Again, an aqueous layer was extracted with toluene (20 ml). The toluene layers were combined, and the solvent was distilled off under reduced pressure, to obtain an oil of the title compound (0.43 g, (S):(R) = 99.9:0.1 from quantitative chiral HPLC analysis).

¹H-NMR (ppm, CDCl₃): δ 1.99 (t, 1H, J=10.5 Hz, CH), 2.14 (dt, 1H, J= 10.5 and 3.0 Hz, CH), 2.31 (s, 3H, NMe), 2.75-2.90 (m, 2H, CH₂), 3.00-3.15 (m, 2H, CH₂), 3.87 (dd, 1H, J= 10.5 and 3.0 Hz, CH), 7.20-7.42 (m, 5H, aromatic).

### Chiral HPLC analysis condition of (S)-1-methyl-3-phenylpiperazine

Column; CHIRALCEL OD-H (4.6×250 mm, manufactured by Daicel Chemical Industries, Ltd.)

Mobile phase; liquid A: isopropanol (containing 0.1% diethylamine)
Liquid B: hexane (containing 0.1% diethylamine)

Elution condition; constant composition of liquid A:liquid B = 5:95

Flow rate; 0.5 ml/min.

Wavelength; 254 nm

Retention time; S-isomer near 12.1 minutes, R-isomer near 13.7 minutes

### <Synthesis of (S)-1-methyl-3-phenylpiperazine Examples 5 to 7>

### Example 5

### N-[(S)-N-benzyloxycarbonylphenylglycyl]sarcosine ethyl ester (Z-Phg-Sar-OEt)

(S)-N-benzyloxycarbonylphenylglycine (5.00 g, 0.0175 mol) was dissolved in dichloromethane (20 ml), and triethylamine (2.44 ml, 0.0175 mol) was added at -10°C or lower. To a solution of ethyl chloroformate (1.67 ml, 0.0175 mol) in dichloromethane (15 ml) was added the above-described solution at -10°C or lower, and the mixture was stirred at the same temperature for 10 minutes to obtain a suspension. Separately, sarcosine ethyl ester hydrochloride (2.82 g, 0.0184 mol) was suspended in dichloromethane (20 ml), triethylamine (2.56 ml, 0.0184 mol) was added at -10°C or lower, the deposit was filtrated and washed with dichloromethane (10 ml), and the filtrate was added to the above-described suspension at -10°C or lower, and the mixture was stirred for 15 minutes, and further, stirred at 0°C for 30 minutes. This reaction mixture was poured into ice water (80 ml), and dichloromethane was once distilled off under reduced pressure. Ethyl acetate (40 ml) was added, insoluble substances were removed by filtration, the filtrate was partitioned, and an organic layer was washed sequentially with 10% hydrochloric acid (15 ml) and saturated sodium bicarbonate water (15 ml), dried over anhydrous magnesium sulfate, then, filtrated, and the solvent was distilled off under reduced pressure. The residue was purified by silica gel column chromatography (developing solvent: hexane:ethyl acetate = 2:1) to obtain 3.87 g(yield 57.4%) of an oil of the title compound.

¹H-NMR(ppm, CDCl₃): δ 1.24 (t, 3H, J=7.0 Hz, CH₃), 2.93 (s, 3H, NMe), 3.98 and 4.27 (2d, 2H, J= 17.2 Hz, CH₂), 4.19 (q, 2H, J= 7.0 Hz, CH₂), 5.04 and 5.01 (2d, 2H, J=12.0 Hz, CH₂), 5.66 (d, 1H, J=7.6 Hz, CH), 6.25 (d, 1H, J=7.2 Hz, NH), 7.20-7.45 (m, 10H, aromatic).

### Example 6

### (S)-1-methyl-3-phenylpiperazine-2,5-dione

An oil of N-[(S)-N-benzyloxycarbonylphenylglycyl]sarcosine ethyl ester (3.8 g) was dissolved in acetic acid (30 ml) 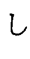 , 5% Pd-C (50% wet product, 0.3 g) was added to cause suspension, and the suspension was stirred in an autoclave under a hydrogen pressure of 5 kg/cm². After 2 hours, Pd-C was removed by filtration, and most of acetic acid was distilled off under reduced pressure. The resultant oil was allowed to stand at 25°C under reduced pressure (0.7 kPa) overnight, then, the residue was purified by silica gel column chromatography (ethyl acetate:acetone = 1:1 to 1:2), the solvent was distilled off, then, the deposited crystal was suspended with heptanes-diethyl ether (1:1, 10 ml), then, the suspension was filtrated to obtain 1.78 g(yield 88%) of the title compound. ¹H-NMR coincided with that obtained in Example 3.

### Example 7

### (S)-1-methyl-3-phenylpiperazine

Under a nitrogen atmosphere, to a suspension of lithium aluminum hydride (0.74 g, 19.5 mmol) in THF (30 ml) was added (S)-1-methyl-3-phenylpiperazine-2,5-dione (1.0 g, 4.9 mmol), then, the mixture was refluxed for 3.5 hours. The mixture was ice-cooled, and ethyl acetate (5 ml), methanol (5 ml) and water (10 ml) were added, and the solvent was once distilled off under reduced pressure. Ethyl acetate (20 ml) and 10% NaOH aqueous solution (10 ml) were added, insoluble substances were removed by filtration through cerite, and an ethyl acetate layer was separated, and the solvent was distilled off under reduced pressure, to obtain an oil of the title compound (0.67 g, 77.6%). ¹H-NMR coincided with that obtained in Example 4.

### Example 8

### (S)-1-methyl-3-phenylpiperazine·oxalate

Into a solution of oxalic acid (0.102 g, 1.13 mmol) in acetone (3 ml) was dropped a solution of (S)-1-methyl-3-phenylpiperazine (0.200 g, 1.14 mmol) in acetone (3 ml). The deposited crystal was filtrated, washed with acetone (3 ml), and dried to obtain 0.275 g (yield 91.1%) of a crystal of the title compound.

m.p. 124 to 127°C (sintered)

¹H-NMR (ppm, D₂O): δ 2.85 (d, 3H, J=3.4 Hz, NMe), 3.15-3.30 (m, 1H, CH), 3.32-3.52 (m, 2H, CH₂), 3.55-3.75 (m, 3H, CH+CH₂), 4.48-4.58 (m, 1H, CH), 7.30-7.50 (m, 5H, aromatic).

### Example 9

### (S)-1-(3-cyano-2-pyridyl)-4-methyl-2-phenylpiperazine oxalate

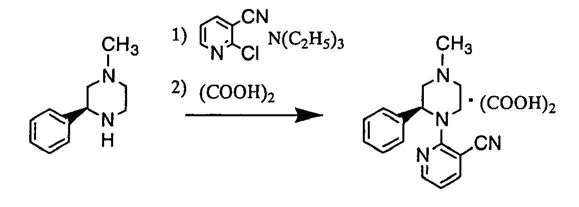

(S)-1-methyl-3-phenylpiperazine (S:R = 97.5:2.5, 2.4 g (12.5 mmol)), N,N-dimethyl-formaldehyde (4.5 g), 2-chloro-3-cyano-pyridine (2.7 g, 19.5 mmol) and triethylamine (1.9 g, 18.8 mmol) were charged, and stirred under nitrogen flow at 120 to 125°C for 17 hours. N,N-dimethylformaldehyde was distilled off at a bath temperature of 90°C under reduced pressure, and thereafter, the mixture was cooled down to 70°C and 3.6 ml was water was flown in. The mixture was further cooled down to 40°C, and pH was controlled to 8.0 with 0.7 ml of 25% sodium hydroxide, then, extraction with 11 ml of ethyl acetate was carried out. An aqueous layer was partitioned and removed, then, an ethyl acetate layer was washed with 2.4 ml of 5% saline, then, 3.6 ml of methanol was flow in, and 1.7 g of oxalic acid bihydrate was added divisionally while keeping temperature at 40°C. After confirmation of crystallization, the mixture was stirred for 1 hour, and further cooled down to 30°C and the mixture was stirred for 1 hour. The crystal was filtrated, and the filtrated residue was washed with 7.7 ml of ethyl acetate-methanol mixed liquid (75:25), and the resultant crystal was dried under reduced pressure at a bath temperature of 40°C, to obtain 3.2 g (yield 66.7%) of the title compound.

### Example 10

### (S)-2-(4-methyl-2-phenylpiperazin-1-yl)-3-pyridinecarbaldehyde

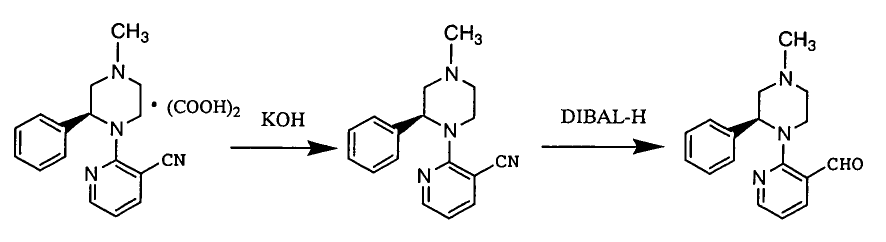

5 ml of toluene, 3.5 ml of water and 0.31 g (5.5 mmol) of potassium hydroxide were charged and stirred, and 1.0 g (2.6 mmol) of 1-(3-cyano-2-pyridyl)-4-methyl-2-phenylpiperazine, oxalate (S-isomer:R-isomer = 96.53:3.47) was added, and the mixture was stirred at an outer bath temperature of 25 to 30°C for 1 hour. A toluene layer was separated and washed with 5 ml of 5% saline, and a toluene solution was dried over 0.3 g of anhydrous magnesium sulfate (stirred for 1 hour at room temperature). Thereafter, magnesium sulfate was filtrated off, and washing with 5 ml of toluene was performed, to obtain a toluene solution of a free body of 1-(3-cyano-2-pyridyl)-4-methyl-2-phenylpiperazine. This was charged into a 100 ml four-necked flask, and cooled down to -60°C in a dry ice bath, then, under nitrogen flow, 4 ml (6.1 mmol) of hydrogenated diisobutylaluminum (DIBAL-H, manufactured by Wako Pure Chemical Industries Ltd.: 25% toluene solution) was dropped from a syringe over a period of 7 minutes (-67 to -52.4°C). The mixture was aged for 2 hours (-64 to -44.1°C) while stirring, and samples were taken and almost completion of the reaction was confirmed by HPLC.

After the reaction, 2 ml of ethanol was dropped while noticing foaming, to decompose excess hydrogenated diisobutylaluminum. Then, sodium hydroxide water (sodium hydroxide 0.85 g/water 5 ml) was added and the mixture was stirred at 30°C for 1 hour, then, a toluene layer was separated, then, dilute sulfuric acid (sulfuric acid 0.42 g/ 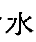 5 ml) was added to this toluene layer, and the mixture was stirred for 1 hour at 45 to 50°C. The mixture was cooled down to 25°C, then, sodium hydroxide water (sodium hydroxide 0.85 g/water 5 ml) was added and the mixture was stirred for 10 minutes, and partitioned, then, a toluene layer was washed with 5 ml of water, and dried over 0.6 g of anhydrous magnesium sulfate. Magnesium sulfate was removed by filtration, then, the bath temperature was set at 40°C and toluene was distilled off under reduced pressure by an evaporator, and further evacuated at the same temperature for 1 hour using a vacuum pump to obtain 0.71 g of the title compound (yield 91.8%, S-isomer:R-isomer = 96.2:3.8).

The R-isomer/S-isomer ratio was measured under the following HPLC analysis conditions.

Column: CHIRALCEL OD-H (0.46 cmφ × 25 cm, manufactured by Daicel Chemical Industries, Ltd.)

Mobile phase; liquid A: 0.1% diethylamine-containing 2-propanol
Liquid B: 0.1% diethylamine-containing n-hexane

Flow rate; liquid A: 0.025 ml/min.
liquid B: 0.475 ml/min (constant composition, constant flow rate)

Column temperature: 40°C

Wavelength; 254 nm

### Example 11

### (S)-1-(3-hydroxymethyl-2-pyridyl)-4-methyl-2-phenylpiperazine

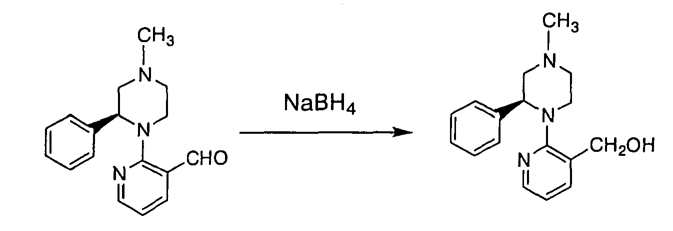

0.65 g (2.2 mmol) of (S)-2-(4-methyl-2-phenylpiperazin-1-yl)-3-pyridinecarbaldehyde obtained in Example 10 was dissolved in 5.3 ml of MeOH, then, the solution was cooled down to 5°C or lower with an ice water bath. 0.2 g (5.3 mmol) of NaBH₄ (manufactured by Wako Pure Chemical Industries Ltd.) was added divisionally over a period of 30 minutes while keeping the temperature at 5°C or lower, and samples were taken for checking the reaction, and stirred for 30 minutes. After confirmation of the reaction by HPLC, 3.5 ml of water was dropped, and 1.7 ml of 35% hydrochloric acid was further dropped over a period of 10 minutes (vigorous foaming). The mixture was further stirred for 5 minutes, then, acidity was confirmed with versatile test paper, then, neutralized with sodium bicarbonate (required 0.7 g). Thereafter, the liquid was distilled at a bath temperature of 40°C under reduced pressure by a rotary evaporator, and 14 ml of toluene and 7 ml of water were added and the mixture was stirred while heating in a bath of 40°C to attain dissolution. Toluene was partitioned, and an aqueous layer was further extracted with 14 ml of toluene twice, and three toluene layers were combined. This toluene layer was washed with 3.5 ml of water, and liquid-partitioned, then, dried over 0.3 g of anhydrous magnesium sulfate (stirring at room temperature for 30 minutes), and magnesium sulfate was removed by filtration, then, toluene was distilled off at a bath temperature of 40°C under reduced pressure by a rotary evaporator, to obtain 0.48 g of the title compound (yield 73.3%, S-isomer:R-isomer = 99.93:0.07 (here, R-isomer is broad), HPLC analysis conditions are the same as in Example 10).

### Example 12

### (S)-mirtazapine

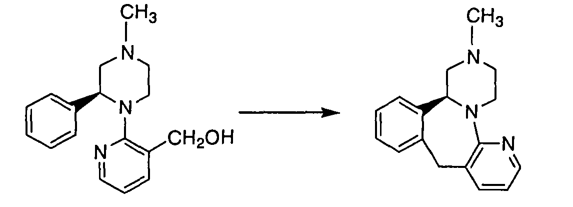

The title compound is obtained from (S)-1-(3-hydroxymethyl-2-pyridyl)-4-methyl-2-phenylpiperazine according to a method described in WO 2005/005410, Example 1a.

### Example 13

### (S)-N-sarcosyl-phenylglycine methyl ester

A crystal (16 g, 41.4 mmol) of (S)-N-[N-benzyloxycarbonylsarcosyl]phenylglycine methyl ester was dissolved in methanol (320 ml), 5% Pd-C (50% wet product, 1.6 g) was added, then, purged with hydrogen, and the mixture was stirred for 2 hours at 23 to 30°C while feeding hydrogen. The catalyst was filtrated, to obtain a methanol solution of (S)-N-sarcosyl-phenylglycine methyl ester. As a result of HPLC analysis shown below, (S)-N-sarcosyl-phenylglycine methyl ester was 77.18% and (S)-1-methyl-3-phenylpiperazine-2,5-dione was 22.54%.

### HPLC analysis condition

Column: CHIRALPAX AD-H (0.46 cmφ × 25 cm, manufactured by Daicel Chemical Industries, Ltd.)

Mobile phase; liquid A: n-hexane
Liquid B: 2-propanol

Flow rate; liquid A: 0.8 ml/min.
liquid B: 0.2 ml/min

Column temperature: 40°C

Wavelength; 210 nm

### Example 14

### (S)-1-methyl-3-phenylpiperazine-2,5-dione

To the methanol solution of (S)-N-sarcosyl-phenylglycine methyl ester obtained in Example 13 was added 2.5 g of phosphoric acid, and the mixture was stirred at 25 to 28°C for 2 hours. Methanol was distilled off under reduced pressure by an evaporator at a bath temperature of 40°C. 50 ml of isopropyl alcohol was added, the mixture was stirred, then, cooled down to 5°C or lower, and the crystal was filtrated to obtain 8.04 g of the title compound (yield from (S)-N-[N-benzyloxycarbonylsarcosyl]phenylglycine methyl ester: 88.2%, S-isomer:R-isomer = 99.94:0.06, HPLC analysis conditions are the same as described in Example 13).

According to the present invention, optically active 1-methyl-3-phenylpiperazine or salt thereof useful for production of pharmaceutical products such as optically active mirtazapine and the like can be produced simply and economically advantageously.

Further, optically active 1-(3-hydroxymethyl-2-pyridyl)-4-methyl-2-phenylpiperazine can be produced from optically active 1-methyl-3-phenylpiperazine while retaining its steric configuration.

## Claims

1. A method of producing optically active 1-methyl-3-phenylpiperazine of the formula (11): 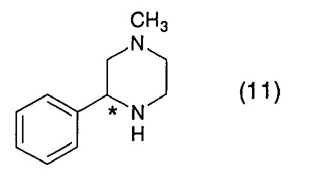 (wherein, a carbon atom with * represents an asymmetrical carbon atom.)
or salt thereof, comprising the following steps 1 to 4: (step 1)
a sarcosine compound of the formula (1): 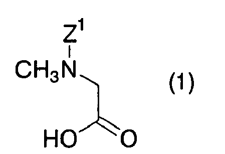 (wherein, Z¹ represents an amino group-protective group.)
or a reactive derivative at its carboxyl group is reacted with an optically active phenylglycine compound of the formula (2): 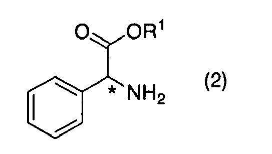 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and * has the same definition as described above.)
to produce an optically active compound of the formula (3): 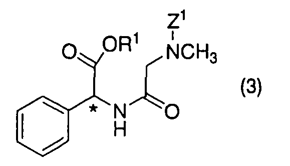 (wherein, Z¹, R¹ and * are as defined above.)
or salt thereof;
(step 2)
the protective group Z¹ is removed from the optically active compound (3) or salt thereof to produce an optically active compound of the formula (4): 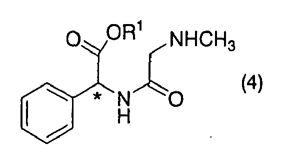 (wherein, marks are as defined above.)
or salt thereof;
(step 3)
the optically active compound (4) or salt thereof is cyclized to produce an optically active 1-methyl-3-phenylpiperazine-2,5-dione of the formula (9): 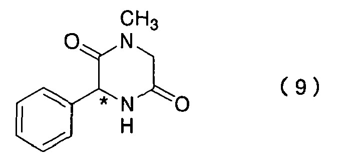 or salt thereof;
(step 4)
the optically active 1-methyl-3-phenylpiperazine-2,5-dione of the formula (9) or salt thereof is reduced to produce optically active 1-methyl-3-phenylpiperazine of the formula (11) or salt thereof.

2. The method according to Claim 1, wherein the step 1 is carried out by reacting a reactive derivative at a carboxyl group of a sarcosine compound of the formula (1) with an optically active phenylglycine compound of the formula (2), or reacting a sarcosine compound of the formula (1) with an optically active phenylglycine compound of the formula (2) in the presence of a condensation agent,
Z¹ is an optionally substituted benzyloxycarbonyl group and removal in the step 2 is carried out by catalytic reduction, or Z¹ is a tert-butoxycarbonyl group and the removal is carried out by an acid treatment,
cyclization in the step 3 is carried out by at least one treatment selected from the group consisting of a heat treatment, pressure reduction treatment, base treatment and acid treatment, and
reduction in the step 4 is carried out by reacting an optically active 1-methyl-3-phenylpiperazine-2,5-dione of the formula (9) or salt thereof with a reducing agent.

3. The method according to Claim 1, wherein the optically active phenylglycine compound of the formula (2) is an S-isomer and the optically active 1-methyl-3-phenylpiperazine of the formula (11) or salt thereof is an S-isomer.

4. A method of producing an optically active 1-methyl-3-phenylpiperazine of the formula (11): 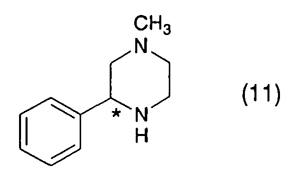 (wherein, a carbon atom with * represents an asymmetrical carbon atom.)
or salt thereof, comprising the following steps 5 to 7 and the step 4:
(step 5)
an optically active phenylglycine compound of the formula (5): 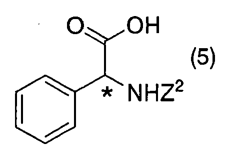 (wherein, Z² represents an amino group-protective group, and * has the same definition as described above.)
or a reactive derivative at its carboxyl group is reacted with a sarcosine compound of the formula (6): 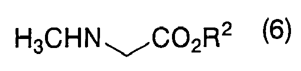 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms.)
to produce an optically active compound of the formula (7): 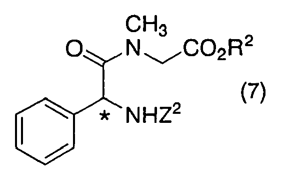 (wherein, Z², R² and * have the same definitions as defined above.)
or salt thereof;
(step 6)
the protective group Z² is removed from an optically active compound of the formula (7) or salt thereof to produce an optically active compound of the formula (8): 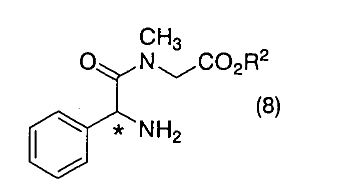 (wherein, R² and * have the same meanings as defined above.)
or salt thereof;
(step 7)
the optically active compound of the formula (8) or salt thereof is cyclized to produce optically active 1-methyl-3-phenylpiperazine-2,5-dione of the formula (9): 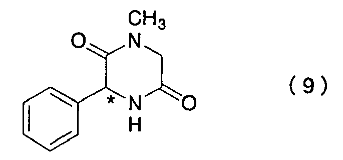 (wherein, * has the same definition as described above.)
or salt thereof;
(step 4)
the optically active 1-methyl-3-phenylpiperazine-2,5-dione of the formula (9) is reduced to produce optically active 1-methyl-3-phenylpiperazine of the formula (11) or salt thereof.

5. The method according to Claim 4, wherein the step 5 is carried out by reacting a reactive derivative at a carboxyl group of an optically active phenylglycine compound of the formula (5) with a sarcosine compound of the formula (6), or reacting an optically active phenylglycine compound of the formula (5) with a sarcosine compound of the formula (6) in the presence of a condensation agent,
the protective group Z² of an optically active compound of the formula (7) or salt thereof is an optionally substituted benzyloxycarbonyl group and removal in the step 6 is carried out by catalytic reduction, or the protective group Z² is a tert-butoxycarbonyl substituent and the removal is carried out by an acid treatment,
cyclization in the step 7 is carried out by at least one treatment selected from the group consisting of a heat treatment, pressure reduction treatment, base treatment and acid treatment, and
reduction in the step 4 is carried out by allowing a reducing agent to act on the optically active 1-methyl-3-phenylpiperazine-2,5-dione of the formula (9) or salt thereof.

6. The method according to Claim 4, wherein the optically active phenylglycine compound of the formula (5) is an S-isomer and the optically active 1-methyl-3-phenylpiperazine of the formula (11) or salt thereof is an S-isomer.

7. A method of producing optically active mirtazapine of the formula (15): 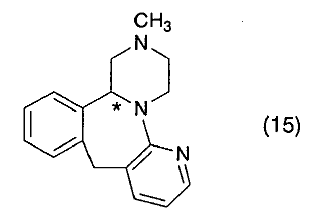 (wherein, a carbon atom with * represents an asymmetrical carbon atom.)
or salt thereof, comprising the following steps 11 to 14:
(step 11)
optically active 1-methyl-3-phenylpiperazine of the formula (11): 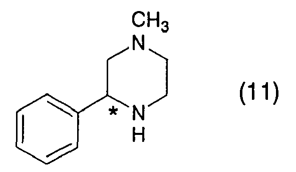 (wherein, * has the same definition as described above.)
or salt thereof is condensed with 2-halogeno-3-cyano-pyridine in the presence of a base to produce optically active 1-(3-cyano-2-pyridyl)-4-methyl-2-phenylpiperazine of the formula (12): 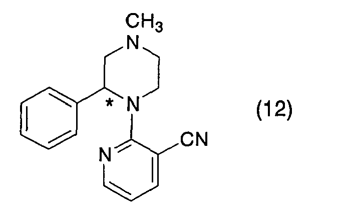 (wherein, * has the same definition as described above.)
or salt thereof;
(step 12)
the optically active 1-(3-cyano-2-pyridyl)-4-methyl-2-phenylpiperazine of the formula (12) or salt thereof is reduced with a reducing agent to produce optically active 2-(4-methyl-2-phenylpiperazin-1-yl)-3-pyridinecarbaldehyde of the formula (13): 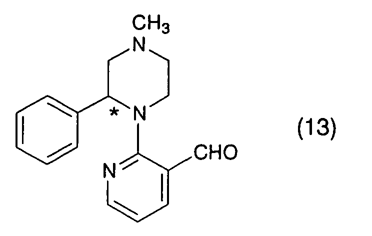 (wherein, * has the same definition as described above.)
or salt thereof;
(step 13)
the optically active 2-(4-methyl-2-phenylpiperazin-1-yl)-3-pyridinecarbaldehyde of the formula (13) or salt thereof is reduced with a reducing agent to produce optically active 1-(3-hydroxymethyl-2-pyridyl)-4-methyl-2-phenylpiperazine of the formula (14): 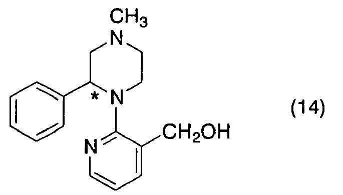 (wherein, * has the same definition as described above.)
or salt thereof;
(step 14)
the optically active 1-(3-hydroxymethyl-2-pyridyl)-4-methyl-2-phenylpiperazine of the formula (14) or salt thereof is cyclized to produce optically active mirtazapine of the formula (15) or salt thereof.

8. The method according to Claim 7, wherein,
in the step 11, the 2-halogeno-3-cyano-pyridine is 2-chloro-3-cyano-pyridine or 2-bromo-3-cyano-pyridine and the base is an organic base,
in the step 12, the reducing agent is hydrogenated diisobutylaluminum, hydrogenated diisopropylaluminum or hydrogenated dihexylaluminum,
in the step 13, the reducing agent is sodium borohydride, sodium cyanoborohydride or lithium borohydride, and
in the step 14, the cyclization is carried out by an acid treatment.

9. The method according to Claim 7, wherein the optically active 1-methyl-3-phenylpiperazine of the formula (11) or salt thereof is an S-isomer, and the optically active mirtazapine of the formula (15) or salt thereof is an S-isomer.

10. The method according to Claim 7, wherein the optically active 1-methyl-3-phenylpiperazine of the formula (11) or salt thereof is obtained by the following steps 1 to 4:
(step 1)
a sarcosine compound of the formula (1): 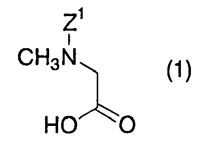 (wherein, Z¹ represents an amino group-protective group.)
or a reactive derivative at its carboxyl group is reacted with an optically active phenylglycine compound of the formula (2): 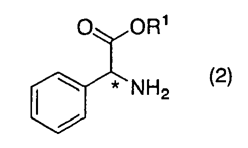 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and * has the same definition as described above.)
to produce an optically active compound of the formula (3): 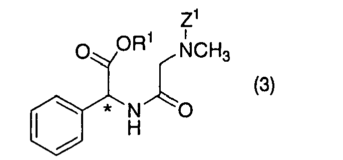 (wherein, Z¹, R¹ and * are as defined above.)
or salt thereof;
(step 2)
the protective group Z¹ is removed from the optically active compound of the formula (3) or salt thereof to produce an optically active compound of the formula (4): 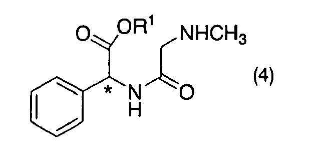 (wherein, R¹ and * have the same definitions as described above.)
or salt thereof;
(step 3)
the optically active compound of the formula (4) or salt thereof is cyclized to produce an optically active 1-methyl-3-phenylpiperazine-2,5-dione of the formula (9): 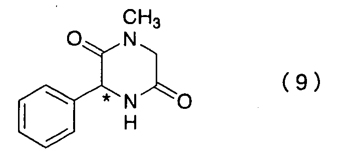 (wherein, * has the same definition as described above.)
or salt thereof;
(step 4)
the optically active 1-methyl-3-phenylpiperazine-2,5-dione of the formula (9) or salt thereof is reduced to produce optically active 1-methyl-3-phenylpiperazine of the formula (11) or salt thereof.

11. The method according to Claim 7, wherein the optically active 1-methyl-3-phenylpiperazine of the formula (11) or salt thereof is obtained by the following steps 5 to 7 and the step 4: (step 5)
an optically active phenylglycine compound of the formula (5): 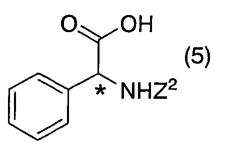 (wherein, Z² represents an amino group-protective group, and * has the same definition as described above.)
or a reactive derivative at its carboxyl group is reacted with a sarcosine compound of the formula (6): 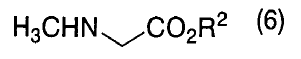 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms.)
to produce an optically active compound of the formula (7): 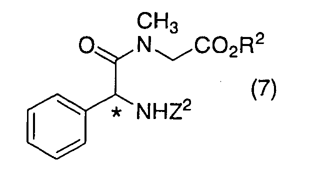 (wherein, Z², R² and * have the same definitions as defined above.)
or salt thereof;
(step 6)
the protective group Z² is removed from the optically active compound of the formula (7) or salt thereof to produce an optically active compound of the formula (8): 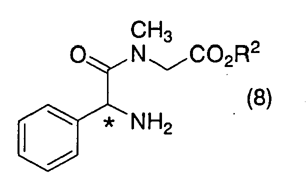 (wherein, R² and * have the same meanings as defined above.)
or salt thereof;
(step 7)
the optically active compound of the formula (8) or salt thereof is cyclized to produce optically active 1-methyl-3-phenylpiperazine-2,5-dione of the formula (9): 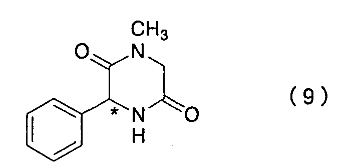 (wherein, * has the same definition as described above.)
or salt thereof;
(step 4)
the optically active 1-methyl-3-phenylpiperazine-2,5-dione of the formula (9) is reduced to produce optically active 1-methyl-3-phenylpiperazine of the formula (11) or salt thereof.

12. A method of producing optically active 1-(3-hydroxymethyl-2-pyridyl)-4-methyl-2-phenylpiperazine of the formula (14) : 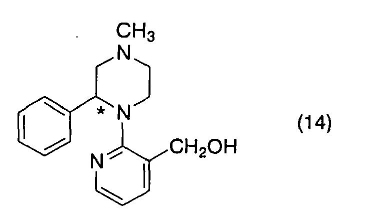 (wherein, a carbon atom with * represents an asymmetrical carbon atom.)
or salt thereof, comprising the following steps 11 to 13:
(step 11)
optically active 1-methyl-3-phenylpiperazine of the formula (11): 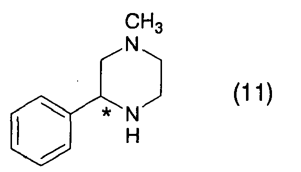 (wherein, * has the same definition as described above.)
or salt thereof is condensed with 2-halogeno-3-cyano-pyridine in the presence of a base to produce optically active 1-(3-cyano-2-pyridyl)-4-methyl-2-phenylpiperazine of the formula (12): 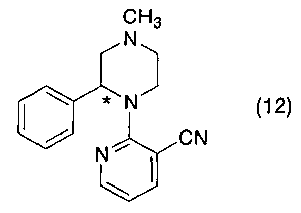 (wherein, * has the same definition as described above.)
or salt thereof;
(step 12)
the optically active 1-(3-cyano-2-pyridyl)-4-methyl-2-phenylpiperazine of the formula (12) or salt thereof is reduced with a reducing agent to produce optically active 2-(4-methyl-2-phenylpiperazin-1-yl)-3-pyridinecarbaldehyde of the formula (13): 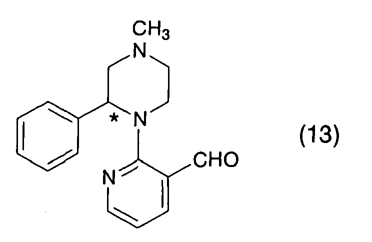 (wherein, * has the same definition as described above.)
or salt thereof;
(step 13)
the optically active 2-(4-methyl-2-phenylpiperazin-1-yl)-3-pyridinecarbaldehyde of the formula (13) or salt thereof is reduced with a reducing agent to produce optically active 1-(3-hydroxymethyl-2-pyridyl)-4-methyl-2-phenylpiperazine of the formula of the formula (14) or salt thereof.

13. Optically active 1-methyl-3-phenylpiperazine of the formula (11): 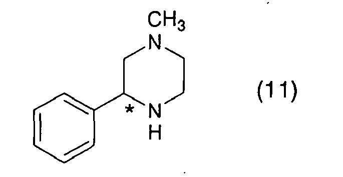 (wherein, a carbon atom with * represents an asymmetrical carbon atom.)
or salt thereof.

14. The compound according to Claim 13, wherein the compound is an S-isomer.

15. 1-methyl-3-phenylpiperazine-2,5-dione of the formula (9'):

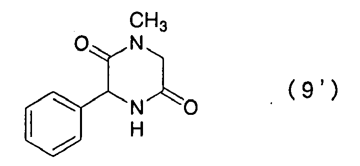

16. The compound according to Claim 15, wherein the compound is an S-isomer.

17. A compound of the formula (3): 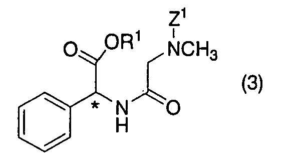 (wherein, Z¹ represents an amino group-protective group, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

18. The compound according to Claim 17, wherein the compound is an S-isomer.

19. A compound of the formula (4): 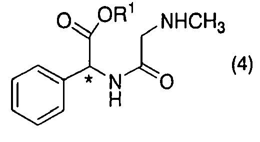 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

20. The compound according to Claim 19 or salt thereof, wherein the compound is an S-isomer.

21. A compound of the formula (7): 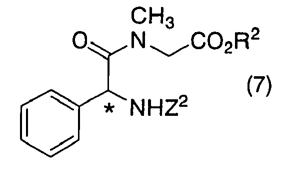 (wherein, Z² represents an amino group-protective group, R² represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

22. The compound according to Claim 21, wherein the compound is an S-isomer.

23. A compound of the formula (8): 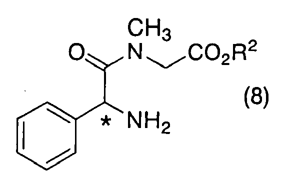 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

24. The compound according to Claim 23, wherein the compound is an S-isomer.

25. Optically active 1-(3-cyano-2-pyridyl)-4-methyl-2-phenylpiperazine of the formula (12): 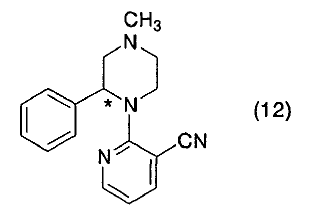 (wherein, a carbon atom with * represents an asymmetrical carbon atom.)
or salt thereof.

26. The compound according to Claim 25, wherein the compound is an S-isomer.

27. Optically active 2-(4-methyl-2-phenylpiperazin-1-yl)-3-pyridinecarbaldehyde of the formula (13): 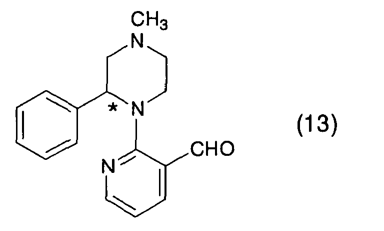 (wherein, a carbon atom with * represents an asymmetrical carbon atom.)
or salt thereof.

28. The compound according to Claim 27, wherein the compound is an S-isomer.

29. A method of producing optically active 1-methyl-3-phenylpiperazine of the formula (11): 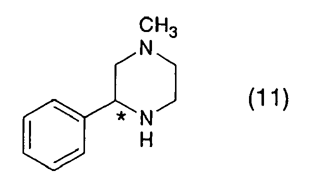 (wherein, * has the same definition as described above.)
or salt thereof, comprising reducing optically active 1-methyl-3-phenylpiperazine-2,5-dione of the formula (9): 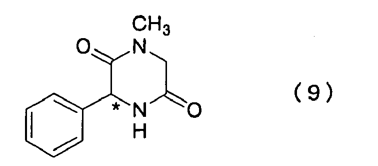 (wherein, * has the same definition as described above.).

30. The method according to Claim 29, wherein reduction is carried out by reacting optically active 1-methyl-3-phenylpiperazine-2,5-dione of the formula (9) or salt thereof with a metal hydride compound.

31. The method according to Claim 30, wherein the metal hydride compound is lithium aluminum hydride.

32. The method according to any one of Claim 29, wherein both the optically active 1-methyl-3-phenylpiperazine-2,5-dione of the formula (9) and the optically active 1-methyl-3-phenylpiperazine of the formula (11) are S-isomers.

33. A method of producing optically active 1-methyl-3-phenylpiperazine-2,5-dione of the formula (9): 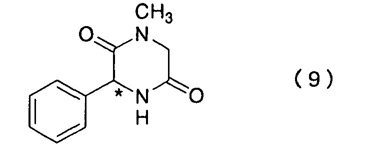 (wherein, * has the same definition as described above.)
, comprising cyclizing the optically active compound of the formula (4): 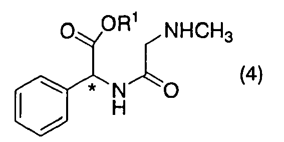 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

34. The method according to Claim 33, wherein cyclization is carried out by at least one treatment selected from the group consisting of a heat treatment, pressure reduction treatment, base treatment and acid treatment.

35. A method of producing an optically active compound of the formula (4): 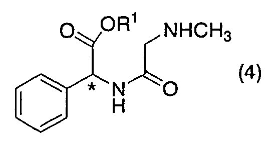 (wherein, R¹ and * have the same definitions as described above.)
or salt thereof, comprising removing the protective group Z¹ from an optically active compound of the formula (3): 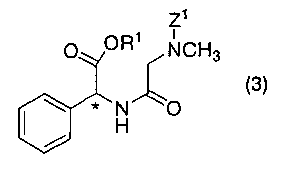 (wherein, Z¹ represents an amino group-protective group, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)

36. A method of producing an optically active compound
of the formula (3): 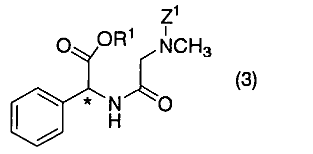 (wherein, marks are as defined above.)
or salt thereof, comprising reacting a sarcosine compound of the formula (1): 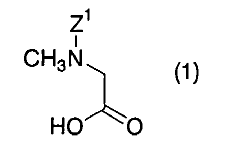 (wherein, Z¹ represents an amino group-protective group.)
or a reactive derivative at its carboxyl group with an optically active phenylglycine compound of the formula (2): 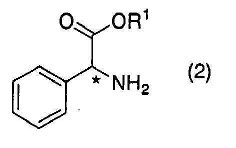 (wherein, R¹ represents an optionally substituted alkyl group having 1 to 6 carbon atoms, an optionally substituted cycloalkyl group having 3 to 7 carbon atoms or an optionally substituted aralkyl group having 7 to 12 carbon atoms, and * has the same definition as described above.).

37. A method of producing optically active 1-methyl-3-phenylpiperazine-2,5-dione of the formula (9): 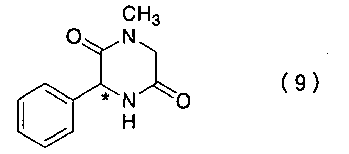 (wherein, * has the same definition as described above.)
, comprising cyclizing optically active compound of the formula (8): 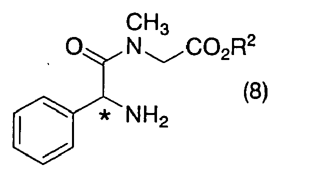 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.)
or salt thereof.

38. The method according to Claim 37, wherein cyclization is carried out by at least one treatment selected from the group consisting of a heat treatment, pressure reduction treatment, base treatment and acid treatment.

39. A method of producing an optically active compound of the formula (8): 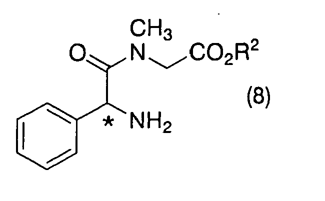 (wherein, R² and * have the same definitions as described above.)
or salt thereof, comprising removing the protective group Z² from an optically active compound of the formula (7): 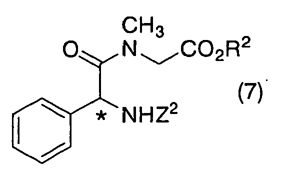 (wherein, Z² represents an amino group-protective group, R² represents an optionally substituted alkyl group having 1 to 6 carbon atoms, an optionally substituted cycloalkyl group having 3 to 7 carbon atoms or an optionally substituted aralkyl group having 7 to 12 carbon atoms, and a carbon atom with * represents an asymmetrical carbon atom.).

40. A method of producing an optically active compound of the formula (7): 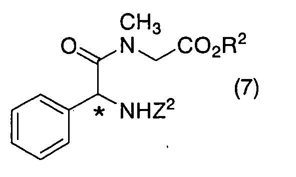 (wherein, Z², R² and * have the same definitions as described above.)
or salt thereof, comprising reacting an optically active phenylglycine compound of the formula (5): 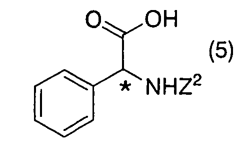 (wherein, Z² represents an amino group-protective group, and a carbon atom with * represents an asymmetrical carbon atom.)
or a reactive derivative at its carboxyl group with a sarcosine compound of the formula (6): 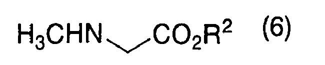 (wherein, R² represents an optionally substituted alkyl group having 1 to 6 carbon atoms, an optionally substituted cycloalkyl group having 3 to 7 carbon atoms or an optionally substituted aralkyl group having 7 to 12 carbon atoms.).

41. A method of producing an optically active 1-(3-hydroxymethyl-2-pyridyl)-4-methyl-2-phenylpiperazine of the formula (14): 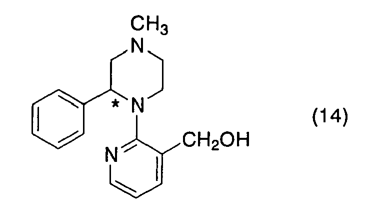 (wherein, * has the same definition as described above.)
or salt thereof, comprising reducing optically active 2-(4-methyl-2-phenylpiperazin-1-yl)-3-pyridinecarbaldehyde of the formula (13): 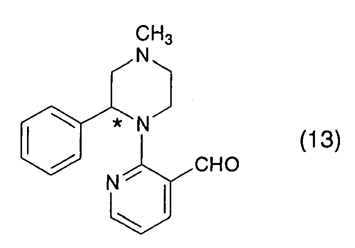 (wherein, a carbon atom with * represents an asymmetrical carbon atom.)
or salt thereof with a reducing agent.

42. The method according to Claim 41, wherein the reducing agent is sodium borohydride.

43. A method of producing an optically active 2-(4-methyl-2-phenylpiperazin-1-yl)-3-pyridinecarbaldehyde of the formula (13): 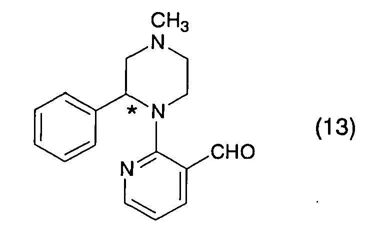 (wherein, * has the same definition as described above.)
or salt thereof, comprising reducing optically active 1-(3-cyano-2-pyridyl)-4-methyl-2-phenylpiperazine of the formula (12): 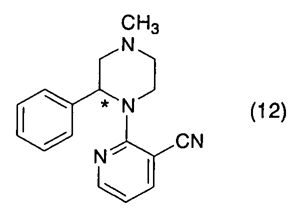 (wherein, a carbon atom with * represents an asymmetrical carbon atom.)
or salt thereof with a reducing agent.

44. The method according to Claim 43, wherein the reducing agent is hydrogenated diisobutylaluminum.

45. A method of producing optically active 1-(3-cyano-2-pyridyl)-4-methyl-2-phenylpiperazine of the formula (12): 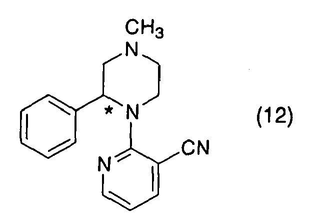 (wherein, * has the same definition as described above.)
or salt thereof, comprising condensing optically active 1-methyl-3-phenylpiperazine of the formula (11): 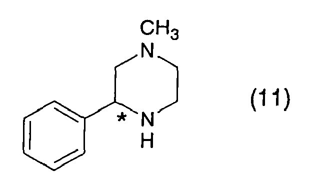 (wherein, a carbon atom with * represents an asymmetrical carbon atom.)
or salt thereof with 2-halogeno-3-cyano-pyridine in the presence of a base.
